# EUROPEAN PATENT APPLICATION

(11) **EP 4 628 013 A1**
(43) Date of publication of application: **08.10.2025**
(21) Application number: 24749604.5
(22) Date of filing: 25.01.2024
(51) Int. Cl.: A61B 5/145

(54) **SENSOR SEALING ASSEMBLY, SENSOR ASSEMBLY, AND PHYSIOLOGICAL SIGN DETECTION DEVICE**

(30) Priority: 31.01.2023 CN 202310145252
(71) Applicant: Huawei Technologies Co., Ltd., Shenzhen, Guangdong 518129 (CN)
(72) Inventor: DONG, Wenxiao, Shenzhen, Guangdong 518129 (CN); SUN, Yuning, Shenzhen, Guangdong 518129 (CN); ZOU, Lin, Shenzhen, Guangdong 518129 (CN); LIU, Xiangyu, Shenzhen, Guangdong 518129 (CN); XIE, Songlin, Shenzhen, Guangdong 518129 (CN); HU, Yifan, Shenzhen, Guangdong 518129 (CN)
(74) Representative: Goddar, Heinz J.
(86) International application number: PCT/CN2024/074011
(87) International publication number: WO 2024/160123

(57) **Abstract**

A sensor sealing component (24), a sensor component (20), and a physiological sign detection apparatus (100) are disclosed. The sensor sealing component (24) includes a needle base (241), a puncture needle (242), a sensor (244), and a sealing bottle (246). The puncture needle (242) is fastened to the needle base (241), and a puncture tip (2421) of the puncture needle (242) protrudes from the needle base (241). The sensor (244) includes an implantation portion (2443), and the implantation portion (2443) of the sensor (244) is located on an inner side of the puncture needle (242). The sealing bottle (246) is detachably fastened to the needle base (241), and hermetically connected to the needle base (241), the sealing bottle (246) has a hollow cavity, and the implantation portion (2443) of the sensor (244) and the puncture tip (2421) of the puncture needle (242) are located in the hollow cavity of the sealing bottle (246). The sensor sealing component (24) can be sterilized independently. In this way, sterilization procedures are simpler and more efficient.

## Description

This application claims priority to Chinese Patent Application No. 202310145252.2, filed with the China National Intellectual Property Administration on January 31, 2023 and entitled "SENSOR SEALING COMPONENT, SENSOR COMPONENT, AND PHYSIOLOGICAL SIGN DETECTION APPARATUS", which is incorporated herein by reference in its entirety.

### TECHNICAL FIELD

This application relates to the field of medical device technologies, and in particular, to a sensor sealing component, a sensor component, and a physiological sign detection apparatus.

### BACKGROUND

Diabetes is a chronic disease that may cause many complications. A conventional diabetes monitoring manner is to use a blood glucose meter to prick a finger to take blood and perform single-point measurement on a glucose concentration in venous blood. Some current minimally invasive blood glucose detection products may implement continuous monitoring for more than 7 days by implanting a biosensor under skin to be in contact with tissue fluids. The minimally invasive blood glucose detection product applies a small sensor component (usually referred to as a patch) to human skin by using an implanter. A sensor of the patch is guided by a puncture needle to subcutaneous tissue, to be in contact with body fluid. The patch transmits analytic data obtained through the sensor to a terminal such as a mobile phone or a display instrument. The sensor used to be implanted into subcutaneous tissue needs to be in a sterile state when being implanted, to ensure user safety.

The sensor component is usually sterilized by using sterilization processes such as steam, irradiation, and epoxyethane. When radiation sterilization is performed on the entire sensor component, radiation exerts adverse impact on an electronic component of the sensor component. Therefore, before radiation sterilization, the electronic component in the sensor component needs to be protected by using a specialized fixture, leading to cumbersome sterilization procedures and reduced efficiency.

### SUMMARY

This application provides a sensor sealing component, a sensor component, and a physiological sign detection apparatus. The sensor sealing component of the sensor component provides a sealed environment for an implantation portion of a sensor and a puncture tip of a puncture needle. The sensor sealing component may be sterilized independently, and then the sterilized sensor sealing component is assembled with another structure such as an electronic part of the sensor component. Therefore, no additional protective fixture is needed when performing radiation sterilization on the sensor sealing component, making sterilization procedures simpler and more efficient.

According to a first aspect, this application provides a sensor sealing component. The sensor sealing component includes a needle base, a puncture needle, a sensor, and a sealing bottle, the puncture needle is fastened to the needle base, and a puncture tip of the puncture needle protrudes from the needle base. The sensor includes an implantation portion, and the implantation portion of the sensor is located on an inner side of the puncture needle. The sealing bottle is detachably fastened to the needle base, and hermetically connected to the needle base, the sealing bottle has a hollow cavity, and the implantation portion of the sensor and the puncture tip of the puncture needle are located in the hollow cavity of the sealing bottle.

In this application, the sensor sealing component provides a sealed environment for the implantation portion of the sensor and the puncture tip of the puncture needle, so that in a sterilization process, the sensor sealing component may be sterilized independently, and then the sterilized sensor sealing component is assembled with another structure such as an electronic part of the sensor component. Therefore, no additional protective fixture is needed when performing radiation sterilization on the sensor sealing component, making sterilization procedures simpler and more efficient. In addition, after sterilization, as a sterile barrier, the sealing bottle may form good isolation protection for the puncture tip of the puncture needle and the implantation portion of the sensor. The puncture tip of the puncture needle and the implantation portion of the sensor are not contaminated for a second time. In subsequent packaging, a sterilized package for ensuring that the sensor is located in a sterile environment does not need to be additionally added to a physiological sign detection apparatus, to reduce costs. In addition, the sensor sealing component has a small volume, and a plurality of sensor sealing assemblies can be sterilized simultaneously through one radiation sterilization procedure, to greatly improve sterilization efficiency and reducing sterilization costs.

In some possible implementations, at least a partial region of the sealing bottle allows ionizing radiation to pass through. In this application, at least a partial region of the sealing bottle allows ionizing radiation to pass through, so that the sensor sealing component can sterilize the puncture tip of the puncture needle and the implantation portion of the sensor through irradiation.

In some possible implementations, the sealing bottle is threadedly connected to, snap-fitted into, or interference-connected to the needle base. In this application, the needle base is threadedly connected to, snap-fitted into, or interference-connected to the sealing bottle, to securely connect the needle base, a first sealing member, a sensor base, a second sealing member, and the sealing bottle, so that the first sealing member is hermetically connected to the needle base and the sensor base, the second sealing member is hermetically connected to the sensor base and the sealing bottle, and the implantation portion of the sensor is located in sealed space formed by using the sensor sealing component. In addition, the implantation portion of the sensor is located in the hollow cavity of the sealing bottle, and the sealing bottle may isolate and protect the implantation portion of the sensor, to avoid damage to a sensing layer of the implantation portion in a subsequent assembly process of the sensor and another component, thereby helping improve reliability and detection accuracy of the physiological sign detection apparatus, and ensuring consistency of batches of sensors in a production process.

In some possible implementations, the sensor sealing component further includes a snap-fit, the snap-fit is fastened to the needle base, and the sealing bottle is snap-fitted into or interference-connected to the snap-fit.

In this application, the sealing bottle is snap-fitted into or interference-connected to the needle base by using the snap-fit, to securely connect the needle base, the first sealing member, the sensor base, the second sealing member, and the sealing bottle, so that the first sealing member is hermetically connected to the needle base and the sensor base, the second sealing member is hermetically connected to the sensor base and the sealing bottle, and the implantation portion of the sensor is located in the sealed space formed by using the sensor sealing component. In addition, the implantation portion of the sensor is located in the hollow cavity of the sealing bottle, and the sealing bottle may isolate and protect the implantation portion of the sensor, to avoid damage to the sensing layer of the implantation portion in the subsequent assembly process of the sensor and another component, thereby helping improve reliability and detection accuracy of the physiological sign detection apparatus.

In some possible implementations, the sealing bottle includes a boss, the boss is located at an end that is of the sealing bottle and that faces away from the needle base, and the sealing bottle is detached from the needle base when the boss is subject to an action force away from the needle base.

For example, a housing cap of the physiological sign detection apparatus is provided with a snap-fitting jaw, and the snap-fitting jaw of the housing cap is snap-fitted with the boss of the sealing bottle. The housing cap is snap-fitted into or interference-connected to the housing. In a process in which the housing cap is detached from the housing, the snap-fitting jaw is snap-fitted with the boss, the snap-fitting jaw drives the sealing bottle to move toward a side away from the sensor component, and the housing cap drives the sealing bottle to be detached from the needle base.

In this application, the housing cap is snap-fitted into or interference-connected to the housing, the sealing bottle is snap-fitted into or interference-connected to the needle base, and the snap-fitting jaw of the housing cap cooperates with the boss of the sealing bottle. When the housing cap is detached from the housing, the sealing bottle may be driven to be detached from the needle base, so that the puncture needle is exposed, to help simplify operation steps of the physiological sign detection apparatus and improve user experience.

In some possible implementations, an inner side of the sealing bottle is provided with an internal thread, an outer side of the needle base is provided with an external thread, the internal thread is engaged with the external thread, the sealing bottle further includes a ratchet, the ratchet is convexly disposed on an outer side surface of the sealing bottle, and the sealing bottle is detached from the needle base when the ratchet is subject to an action force opposite to a rotation direction of the internal thread of the sealing bottle.

For example, the housing cap of the physiological sign detection apparatus is threadedly connected to the housing. The housing cap is provided with an internal thread, the housing is provided with an external thread, and the internal thread of the housing cap is engaged with the external thread of the housing. A rotation direction of the internal thread of the housing cap is the same as a rotation direction of the internal thread of the sealing bottle. In addition, the housing cap is further provided with a pawl, and the pawl of the housing cap and the ratchet of the sealing bottle are arranged in coordination. In a process in which the housing cap is screwed on the housing, the pawl is slidably connected to the ratchet. In other words, in the process in which the housing cap is screwed on the housing, the pawl does not push the ratchet to move, to avoid a case in which the sealing bottle is loose relative to the needle base when the housing cap is assembled to the housing. In a process in which the housing cap is detached from the housing, the pawl is snap-fitted into the ratchet. A rotation direction of the housing cap when the housing cap is detached from the housing is opposite to a rotation direction of the internal thread of the sealing bottle, so that the housing cap can simultaneously push the sealing bottle to rotate. Because the sealing bottle is threadedly connected to the needle base, the sealing bottle can be screwed off relative to the needle base, so that the sealing bottle is detached from the needle base.

In this application, the housing cap is threadedly connected to the housing, the sealing bottle is threadedly connected to the needle base, and the pawl of the housing cap cooperates with the ratchet of the sealing bottle. When the housing cap is detached from the housing, the sealing bottle may be pushed to be detached from the needle base, so that the puncture needle and the sensor are exposed, to help simplify operation steps of the physiological sign detection apparatus, and improve use experience of the user.

In some possible implementations, the sensor sealing component further includes a sensor base, the sensor is fastened to the sensor base, the sensor base is located between the needle base and the sealing bottle, an end of the needle base passes through the sensor base, the sealing bottle is hermetically connected to the sensor base, and the needle base is hermetically connected to the sensor base.

In this application, the sealing bottle is hermetically connected to the sensor base, and the needle base is hermetically connected to the sensor base, so that the implantation portion of the sensor is located in the sealed space formed by using the sensor sealing component. In addition, the implantation portion of the sensor is located in the hollow cavity of the sealing bottle, and the sealing bottle may isolate and protect the implantation portion of the sensor, to avoid damage to the sensing layer of the implantation portion in the subsequent assembly process of the sensor and another component, thereby helping improve reliability and detection accuracy of the physiological sign detection apparatus.

In some possible implementations, the sensor and the sensor base are integrally formed by using an in-mold injection molding process, and a region that is of the sensor and that is in contact with the sensor base is covered with a heat insulation layer. In this application, the sensor and the sensor base are integrally formed by using the in-mold injection molding process, and the region that is of the sensor and that is in contact with the sensor base is covered with the heat insulation layer, to reduce heat conduction in a process of performing in-mold injection molding on the sensor and the sensor base, and prevent heat from being transferred from a fastening portion of the sensor to the implantation portion, so as to prevent an enzyme at the sensing layer of the implantation portion from being deactivated due to a high temperature, thereby helping improve detection accuracy of the physiological sign detection apparatus.

In some possible implementations, the sensor sealing component further includes a first sealing member and a second sealing member, the first sealing member is hermetically connected to the needle base and the sensor base, and the second sealing member is hermetically connected to the sensor base and the sealing bottle.

In this application, the first sealing member is hermetically connected to the needle base and the sensor base, and the second sealing member is hermetically connected to the sensor base and the sealing bottle, so that the implantation portion of the sensor is located in the sealed space formed by using the sensor sealing component. The sealing bottle may isolate and protect the implantation portion of the sensor, to avoid damage to a sensing layer of the implantation portion in a subsequent assembly process of the sensor and another component, thereby helping improve reliability and detection accuracy of the physiological sign detection apparatus, and ensuring consistency of batches of sensors in a production process.

In some possible implementations, the sensor sealing component further includes an upper cover, the sensor is snap-fitted into the upper cover, the upper cover is located between the needle base and the sealing bottle, an end of the needle base passes through the upper cover, the needle base is hermetically connected to the upper cover, and the sealing bottle is hermetically connected to the upper cover.

In this application, a sensor sealing component includes the upper cover, and the upper cover only needs to be provided with a second through hole that allows a guiding portion of the needle base to pass through. A hole diameter of the second through hole is smaller. In addition, the needle base is fastened to the upper cover by being securely connected to the sealing bottle. Assembly between the upper cover and the needle base is simpler, and a lower cover does not need to be provided with a structure for being snap-fitted into the sensor sealing component, to help simplify a structure of the lower cover.

In some possible implementations, the upper cover has a second through hole, the upper cover includes a blocking flange, the blocking flange is disposed around the second through hole, and the sensor is stuck on the blocking flange.

For example, the blocking flange has accommodation space and a blocking groove, and the blocking groove communicates with the accommodation space and the second through hole.

In some possible implementations, the upper cover further includes a first positioning member, the first positioning member is spaced apart from the blocking flange, and the sensor is snap-fitted into the first positioning member. For example, the first positioning member is spaced apart from the blocking flange, and the first positioning member is disposed opposite to the accommodation space of the blocking flange. The first positioning member has a limiting groove. An extension direction of the limiting groove is the same as an extension direction of the blocking groove, and both the limiting groove and the blocking groove are located or approximately located in a same straight line. The fastening portion of the sensor is snap-fitted into the blocking groove of the cover, and a tail end of the connection portion is snap-fitted into the limiting groove of the cover.

In this application, the blocking flange and the first positioning member position and limit the sensor, and the sensor can slightly move in the blocking groove and the limiting groove, to prevent the sensor from being stuck or worn out.

According to a second aspect, this application further provides a sensor component. The sensor component includes an upper cover, a lower cover, and the sensor sealing component according to any one of the foregoing items. The upper cover is fastened to the lower cover, the lower cover has a first through hole, the upper cover has a second through hole, the second through hole directly faces and communicates with the first through hole, and the sensor sealing component runs through the first through hole and the second through hole, and is snap-fitted into the lower cover and/or the upper cover.

In this application, the circuit board and the lower cover form an integral mechanical part by using an adhesive process, so that the circuit board and a bottom plate of the lower cover are stably fastened, to avoid a collision caused by movement of the circuit board relative to the lower cover in a transportation process of the physiological sign detection apparatus, and help improve reliability of the physiological sign detection apparatus.

In some possible implementations, the sensor component further includes a battery, a connector, and a circuit board, accommodation space is formed between the upper cover and the lower cover, the circuit board, the connector, the battery, and a connection portion of a sensor is located in the accommodation space, the circuit board is fastened to the lower cover or the upper cover, the battery and the connector are fastened to the circuit board and are electrically connected to the circuit board, and the sensor of the sensor sealing component is electrically connected to the connector.

According to a third aspect, this application further provides a sensor component. The sensor component includes a circuit board, a connector, and the sensor sealing component according to any one of the foregoing items. The circuit board is arranged on a circumferential side of the sensor sealing component, the connector is fastened to the circuit board and is electrically connected to the circuit board, and a sensor of the sensor sealing component is electrically connected to the connector.

In this application, in the sensor component, the sensor sealing component is arranged in the middle, the circuit board is arranged around the sensor sealing component, the battery, the connector, and another component are arranged on a circumferential side of the sensor sealing component, and a plurality of components of the sensor component are arranged compactly, to facilitate miniaturization.

In some possible implementations, the connector includes a connecting groove, a plurality of first spring plates, and a plurality of second spring plates, the connecting groove includes a first groove wall and a second groove wall that are disposed opposite to each other, the plurality of first spring plates are convexly disposed on the first groove wall, the plurality of second spring plates are convexly disposed on the second groove wall, and the connection portion of the sensor is snap-fitted into the connecting groove of the connector and is electrically connected to the plurality of first spring plates and/or the plurality of second spring plates.

In this application, at least a part of the plurality of first spring plates of the connector is convexly disposed on the first groove wall of the connecting groove, and at least a part of the plurality of second spring plates is convexly disposed on the second groove wall of the connecting groove. A conductive layer on a surface of the sensor can be electrically connected to the plurality of first spring plates and the plurality of second spring plates only by snap-fitting the sensor into the connecting groove of the connector. Assembly between the sensor and the connector is simpler, and an electrical connection between the sensor and the connector is more stable. In addition, the sensor can maintain a plate-like structure, and does not need to be folded additionally due to an electrical connection requirement, so that the sensor has high reliability. In addition, because spring plates are convexly disposed on groove walls on two sides of the connecting groove, the connector has more contacts configured to be electrically connected to the sensor, to reduce an arrangement area of the first spring plate and the second spring plate, thereby reducing a volume of the connector, helping miniaturize the sensor component. In addition, miniaturization of the sensor component also helps miniaturize the physiological sign detection apparatus.

In some possible implementations, the first spring plate and the second spring plate each are formed by bending a metal plate, a plate surface of the first spring plate is in contact with the sensor, and a plate surface of the second spring plate is in contact with the sensor.

In this application, the plate surface of the first spring plate is in contact with the sensor, and the plate surface of the second spring plate is in contact with the sensor. The sensor is in face contact with the first spring plate and the second spring plate, a contact area is large, the sensor is subject to an even force, and the sensor is not easily damaged. In addition, when a tail end of the connection portion of the sensor is snap-fitted into a first positioning member, the tail end may move slightly in a limiting groove. Therefore, the connection portion of the sensor is electrically connected to an abutting portion of the first spring plate and an abutting portion of the second spring plate, and a position of the connection portion of the sensor may be slightly adjusted to implement self-adaptation, to better abut against the first spring plate and the second spring plate, so that a connecting position of the sensor is subject to an even force, thereby ensuring that the sensor is stably connected to the first spring plate and the second spring plate, to reduce fatigue caused by an uneven force applied to the first spring plate and the second spring plate for a long period of time.

According to a fourth aspect, this application further provides a physiological sign detection apparatus. The physiological sign detection apparatus includes an implanter and the sensor component according to any one of foregoing items. The sensor component is detachably fastened to the implanter, and the implanter is configured to push the sensor component to an organism.

In this application, the physiological sign detection apparatus is an integral apparatus. The sensor component is mounted on an inner side of the implanter in advance and then packaged. A user does not need to assemble the implanter and the sensor component in a use process. The user only needs to open one package, and attach the physiological sign detection apparatus to the target monitoring position on the organism. The sensor component is pushed to the target monitoring position by using the implanter of the physiological sign detection apparatus. The integral physiological sign detection apparatus helps simplify steps of user operations, and can avoid a biological contamination risk caused in an assembly process of the user while facilitating the user operations. In addition, the physiological sign detection apparatus needs only one package, and the sensor component and the implanter do not need to be separately packaged, to help reduce product costs and reduce packages and waste.

### BRIEF DESCRIPTION OF DRAWINGS

FIG. 1 is a diagram of a physiological sign detection apparatus in some application scenarios according to this application;
FIG. 2 is a diagram of a structure of the physiological sign detection apparatus shown in FIG. 1 in some embodiments;
FIG. 3 shows a partial cross-sectional structure after the physiological sign detection apparatus shown in FIG. 2 is cut along A-A;
FIG. 4 is a diagram of a structure of a sensor component shown in FIG. 2 in some embodiments;
FIG. 5 is a diagram of a partial exploded structure of the sensor component shown in FIG. 4;
FIG. 6 is a diagram of a partial exploded structure of a sensor sealing component shown in FIG. 5;
FIG. 7A is a diagram of an assembly structure of a sensor and a sensor base that are shown in FIG. 6 in some embodiments;
FIG. 7B is a diagram of a structure of the sensor shown in FIG. 7A;
FIG. 8 is a diagram of an assembly structure of a sensor and a sensor base that are shown in FIG. 6 in some other embodiments;
FIG. 9 is a diagram of an exploded structure of the structure shown in FIG. 8;
FIG. 10 is a diagram of an assembly structure of a puncture needle and a needle base that are shown in FIG. 6;
FIG. 11 is a diagram of a structure of a sealing bottle shown in FIG. 6 from another perspective;
FIG. 12 is a diagram of a cross-sectional structure after a sensor sealing component shown in FIG. 5 is cut along B-B;
FIG. 13 is a diagram of a sterilization tooling structure of a sensor sealing component shown in FIG. 5;
FIG. 14 is a diagram of a partial structure of the sensor component shown in FIG. 4;
FIG. 15 is a diagram of an assembly structure of a sensor and a connector that are shown in FIG. 14;
FIG. 16 is a diagram of a cross-sectional structure after the sensor component shown in FIG. 4 is cut along C-C in some embodiments;
FIG. 17 is a diagram of a cross-sectional structure after the sensor component shown in FIG. 4 is cut along D-D in some embodiments;
FIG. 18 is a diagram of a structure of a sensor component shown in FIG. 2 in some other embodiments;
FIG. 19 is a diagram of a partial exploded structure of the sensor component shown in FIG. 18;
FIG. 20 is a diagram of a partial exploded structure of the sensor sealing component shown in FIG. 19;
FIG. 21 is a diagram of a structure of an upper cover shown in FIG. 20 from another perspective;
FIG. 22 is a diagram of a cross-sectional structure after a sensor sealing component shown in FIG. 19 is cut along E-E;
FIG. 23 is a diagram of a sterilization tooling structure of a sensor sealing component shown in FIG. 19;
FIG. 24A is a diagram of a structure of a connector shown in FIG. 19 in some embodiments;
FIG. 24B is a diagram of a structure of the connector shown in FIG. 24A from another perspective;
FIG. 25 is a diagram of an exploded structure of the connector shown in FIG. 24B;
FIG. 26 is a diagram of a partial structure of the sensor component shown in FIG. 18;
FIG. 27 is a diagram of a cross-sectional structure after the sensor component shown in FIG. 18 is cut along F-F;
FIG. 28 is a diagram of a structure of a sensor component shown in FIG. 2 in some other embodiments;
FIG. 29A is a diagram of a structure of a needle base of the sensor component shown in FIG. 28;
FIG. 29B is a diagram of an assembly structure of a snap-fit and a needle base in some embodiments;
FIG. 30 is a diagram of a structure of a sealing bottle shown in FIG. 28 from another perspective;
FIG. 31 is a diagram of a structure of the sensor sealing component shown in FIG. 28;
FIG. 32 is a diagram of a cross-sectional structure after the sensor component shown in FIG. 28 is cut along G-G; and
FIG. 33 is a diagram of a cross-sectional structure after the sensor component shown in FIG. 28 is cut along H-H.

### DESCRIPTION OF EMBODIMENTS

The following describes the technical solutions in embodiments of this application with reference to the accompanying drawings. In descriptions of embodiments of this application, unless otherwise specified, "/" indicates "or". For example, A/B may indicate A or B. The term "and/or" in this specification merely describes an association relationship for describing associated objects, and indicates that three relationships may exist. For example, A and/or B may indicate the following three cases: Only A exists, both A and B exist, and only B exists. In addition, in the descriptions of embodiments of this application, "a plurality of" means two or more.

In the following, terms such as "first" and "second" are used only for description purposes, and cannot be understood as implying or implying relative importance or implicitly indicating a quantity of indicated technical features. Therefore, a feature limited by "first" or "second" may explicitly or implicitly include one or more features.

Orientation terms mentioned in embodiments of this application, for example, "inside", "outside", "side surface", "top", and "bottom", are merely directions in the accompanying drawings. Therefore, the orientation terms are used to better and more clearly describe and understand embodiments of this application, instead of indicating or implying that a specified apparatus or element needs to have a specific orientation, and be constructed and operated in the specific orientation. Therefore, this cannot be understood as a limitation on embodiments ofthis application.

In the description of embodiments of this application, it should be noted that, unless otherwise specified and limited, terms "installation", "connecting", "connection", and "disposed on ..." should be understood in a broad sense. For example, "connection" may be a detachable connection, or may be an undetachable connection, or may be a direct connection, or may be an indirect connection by using an intermediate medium. "Fastening" means that two parties are connected to each other and a relative position relationship remains unchanged after the two parties are connected to each other.

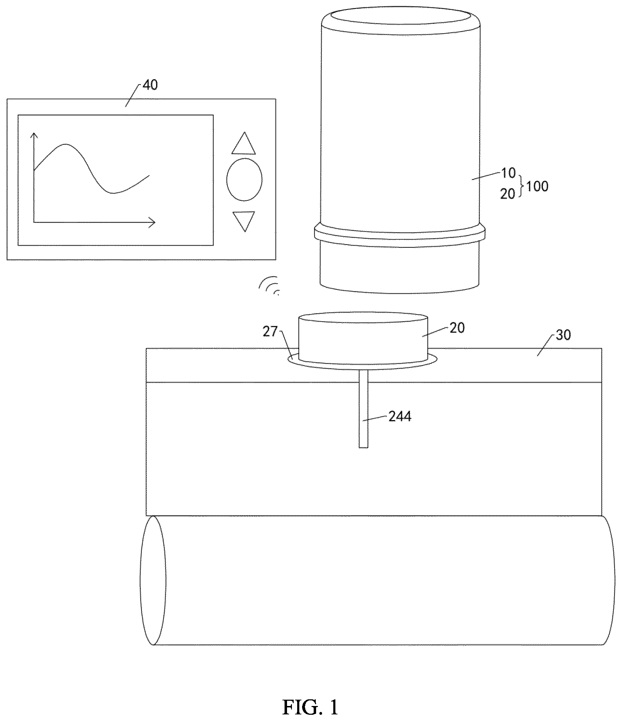

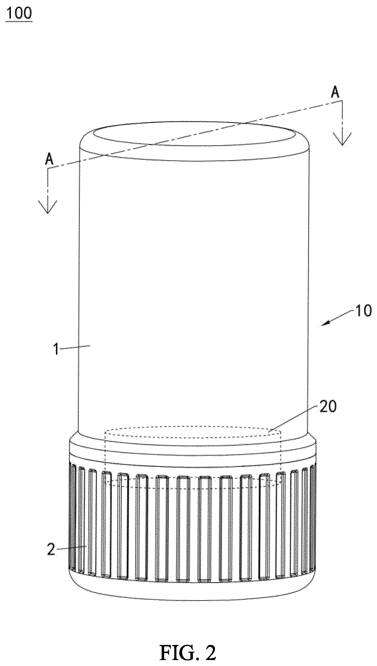

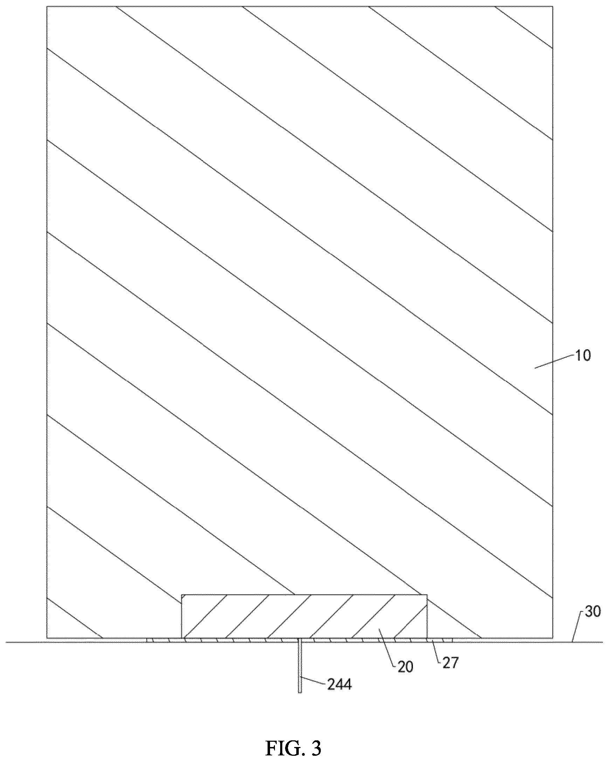

FIG. 1 is a diagram of a physiological sign detection apparatus 100 in some application scenarios according to this application. FIG. 2 is a diagram of a structure of the physiological sign detection apparatus 100 shown in FIG. 1 in some embodiments. FIG. 3 shows a partial cross-sectional structure after the physiological sign detection apparatus 100 shown in FIG. 2 is cut along A-A.

In some embodiments, the physiological sign detection apparatus 100 may include an implanter 10 and a sensor component 20. The sensor component 20 is detachably mounted on an inner side of the implanter 10. The implanter 10 is configured to push the sensor component 20 to a target monitoring position (for example, skin of a belly or a fat-rich part of an arm of the organism 30) on an organism 30. The sensor component 20 may include a sensor 244 and a bonding member 27. After the implanter 10 pushes away the sensor component 20, the sensor component 20 is fastened to the organism 30 by using the bonding member 27 at the bottom of the sensor component 20. A part of the sensor 244 of the sensor component 20 extends from the sensor component 20 and is implanted into subcutaneous tissue of skin of the organism 30, to collect and transfer blood information, so that the sensor 244 can detect and quantify one or more physiological target substances such as blood glucose, lactic acid, uric acid, dissolved oxygen, hydrogen peroxide, and ions. The sensor component 20 may transmit information to a terminal device 40 in a short-range transmission manner such as Bluetooth or a short-range wireless communication technology (Near Field Communication, NFC). The terminal device 40 may be an electronic device with a display, for example, a display instrument, a mobile phone, or an electronic handwatch. After the sensor 244 is implanted into the organism 30, setting is performed by using an application (Application, APP) on the terminal device 40, to start detection of the physiological target substance such as blood glucose. The sensor component 20 transfers collected physiological target substance data to the terminal device 40. The terminal device 40 is configured to observe information such as a concentration of the physiological target substance, to detect and manage a physical and chemical indicator such as blood glucose. The sensor component 20 is a consumable, and one sensor component 20 may be used for at least 14 consecutive days.

In some embodiments, the physiological sign detection apparatus 100 is an integral apparatus. The sensor component 20 is mounted on an inner side of the implanter 10 in advance and then packaged. A user does not need to assemble the implanter 10 and the sensor component 20 in a use process. The user only needs to open one package, and attach the physiological sign detection apparatus 100 to the target monitoring position on the organism 30. The sensor component 20 is pushed to the target monitoring position by using the implanter 10 of the physiological sign detection apparatus 100. The integral physiological sign detection apparatus 100 helps simplify steps of user operations, and can avoid a biological contamination risk caused in an assembly process of the user while facilitating the user operations. In addition, the physiological sign detection apparatus 100 needs only one package, and the sensor component 20 and the implanter 10 do not need to be separately packaged, to help reduce product costs and reduce packages and waste.

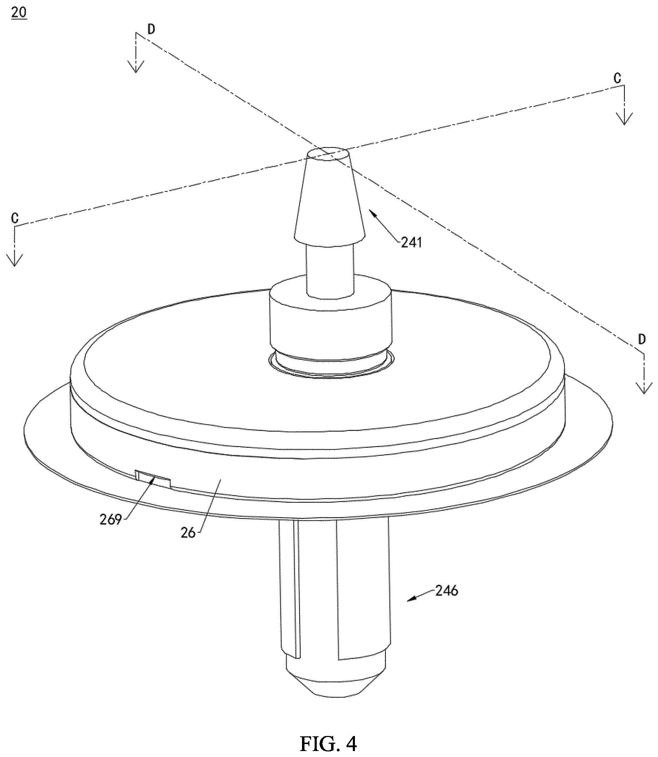

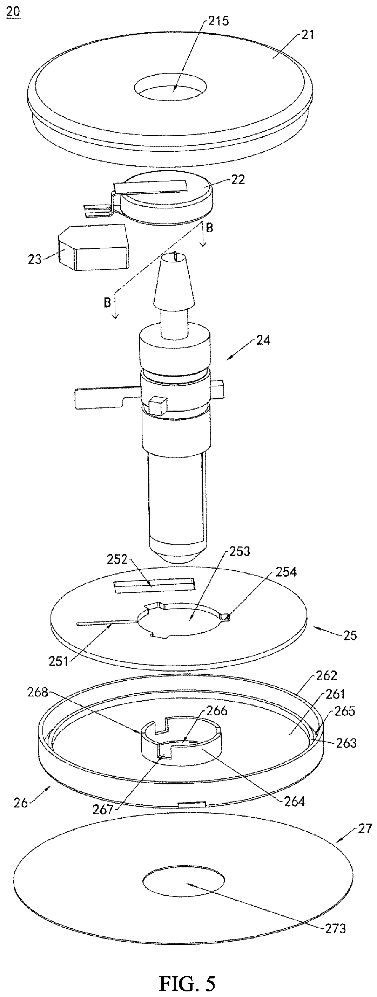

FIG. 4 is a diagram of a structure of the sensor component 20 shown in FIG. 2 in some embodiments. FIG. 5 is a diagram of a partial exploded structure of the sensor component 20 shown in FIG. 4.

In some embodiments, the sensor component 20 may include an upper cover 21, a battery 22, a connector 23, a sensor sealing component 24, a circuit board (Printed Circuit Board, PCB) 25, a lower cover 26, and a bonding member 27. Both the upper cover 21 and the lower cover 26 may be made of engineering plastic such as polycarbonate or acrylonitrile butadiene styrene. Electronic elements such as a chip, a capacitor, a resistor, a near-range wireless communication (Near Field Communication, NFC) component, or a Bluetooth component may be disposed on the circuit board 25. A snap-fitting hook groove 269 may exist on an outer side surface of the lower cover 26 of the sensor component 20, the implanter 10 is provided with a snap-fitting hook, and the snap-fitting hook of the implanter 10 is snap-fitted into the snap-fitting hook groove 269 of the lower cover 26, so that the sensor component 20 is detachably fastened to the implanter 10. After the sensor component 20 is stuck on the organism 30 (as shown in FIG. 1), the implanter 10 can be detached from the sensor component 20.

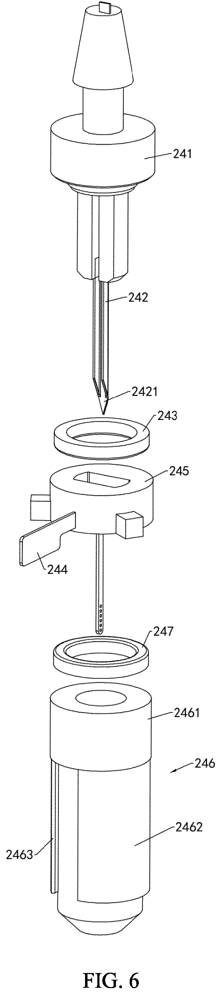

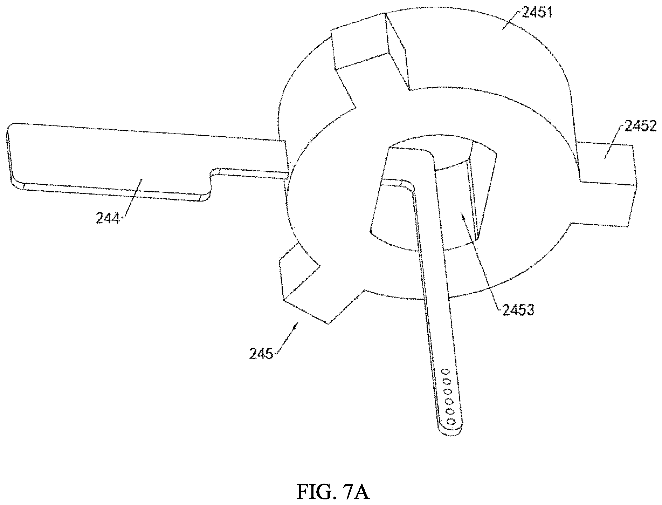

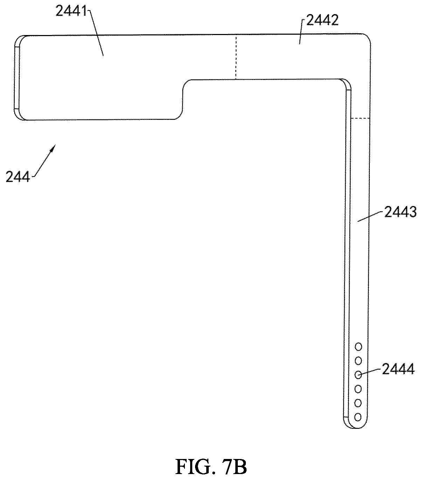

FIG. 6 is a diagram of a partial exploded structure of the sensor sealing component 24 shown in FIG. 5. FIG. 7A is a diagram of an assembly structure of a sensor 244 and a sensor base 245 that are shown in FIG. 6 in some embodiments. FIG. 7B is a diagram of a structure of the sensor 244 shown in FIG. 7A.

In some embodiments, the sensor sealing component 24 may include a needle base 241, a puncture needle 242, a first sealing member 243, a sensor 244, a sensor base 245, a second sealing member 247, and a sealing bottle 246. The sensor 244 is fastened to the sensor base 245. The sensor 244 may include a connection portion 2441, a fastening portion 2442, and an implantation portion 2443. The fastening portion 2442 is connected to the implantation portion 2443 and the connection portion 2441. The connection portion 2441 of the sensor 244 is configured to be electrically connected to the connector 23 (as shown in FIG. 5), the sensor base 245 is fastened to the fastening portion 2442 of the sensor 244, and the implantation portion 2443 of the sensor 244 is configured to be punctured into the organism 30.

The sensor 244 may include a substrate, an electrode layer (not shown in FIG. 7B), and a sensing layer 2444. For example, a material of the substrate may be polyethylene terephthalate (Polyethylene terephthalate, PET). The electrode layer is fastened to a surface of the substrate. The electrode layer may be an electrode system (for example, two electrodes or a plurality of electrodes) formed on the surface of the substrate by using a process such as ink-jet printing or silk-screen printing. The electrode layer may include a first part located in the implantation portion 2443, a second part located in the fastening portion 2442, and a third part located in the connection portion 2441. The second part of the electrode layer is connected to the first part of the electrode layer and the third part of the electrode layer. The sensing layer 2444 is fastened to a partial region in the first part of the electrode layer. In other words, the sensing layer 2444 is located in the implantation portion 2443. The sensing layer 2444 may be formed by applying a sensing reagent to a surface of the first part of the electrode layer through ink-jet printing or drop-casting. The sensing reagent may be a mixed solution including a bioproteinase (for example, glucose oxidase), an electron transfer medium, and a cross-linking agent. After printing of the sensor 244 is completed, a shape may be formed through femtosecond or picosecond laser cutting.

In some embodiments, the sensor 244 and the sensor base 245 are integral mechanical parts. For example, the sensor 244 and the sensor base 245 are integrally formed by using an in-mold injection molding process. A region that is of the sensor 244 and that is in contact with the sensor base 245 may be covered with a heat insulation layer. For example, a surface of the fastening portion 2442 of the sensor 244 may be covered with a heat insulation layer. The heat insulation layer may be formed by using a process such as an ink-jet printing process or a silk-screen printing process.

In this embodiment, the sensor 244 and the sensor base 245 are integrally formed by using the in-mold injection molding process, and the region that is of the sensor 244 and that is in contact with the sensor base 245 is covered with the heat insulation layer, to reduce heat conduction in a process of performing in-mold injection molding on the sensor 244 and the sensor base 245, and prevent heat from being transferred from the fastening portion 2442 of the sensor 244 to the implantation portion 2443, so as to prevent an enzyme at the sensing layer 2444 of the implantation portion 2443 from being deactivated due to a high temperature, thereby helping improve detection accuracy of the physiological sign detection apparatus 100.

In some embodiments, the sensor base 245 may include a body 2451, a blocking block 2452, and a mounting through hole 2453. The sensor 244 may be fastened to the body 2451 of the sensor base 245. The blocking block 2452 is fastened to an outer circumferential edge of the body 2451. There may be one or more blocking blocks 2452, and a plurality of blocking blocks 2452 are spaced apart around a circumferential direction of the body 2451. For example, there may be three blocking blocks 2452. The mounting through hole 2453 passes through the body 2451, and the mounting through hole 2453 may be a strip hole with radians at two ends. The sensor base 245 may be an integral mechanical part. The sensor base 245 may be made of engineering plastic such as polycarbonate or acrylonitrile butadiene styrene.

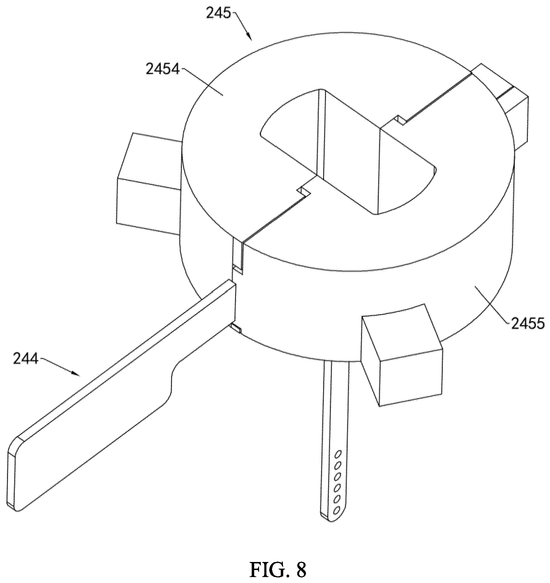

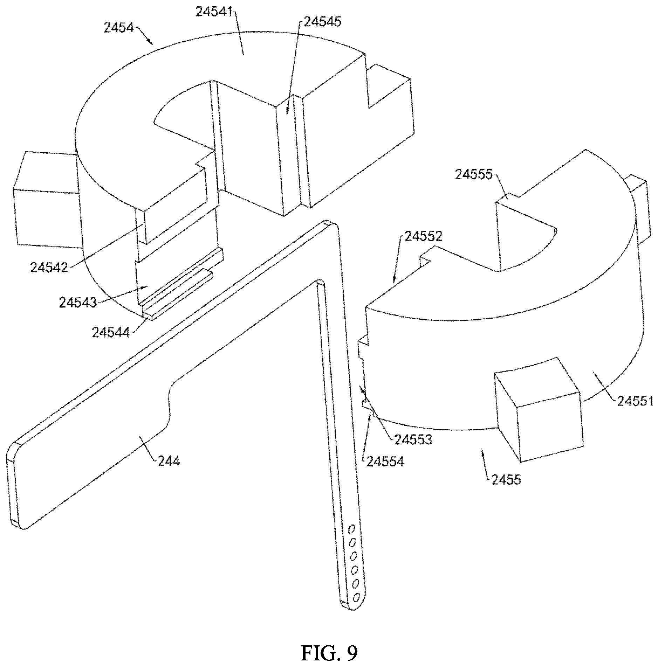

FIG. 8 is a diagram of an assembly structure of the sensor 244 and the sensor base 245 that are shown in FIG. 6 in some other embodiments. FIG. 9 is a diagram of an exploded structure of the structure shown in 8. This embodiment may include most of the technical solutions in the foregoing embodiments. The following mainly describes differences between this embodiment and the foregoing embodiments, and most same content of this embodiment and the foregoing embodiments is not described again.

In some embodiments, the sensor base 245 may include a first part 2454 and a second part 2455. The first part 2454 is snap-fitted into the second part 2455, and the fastening portion 2442 of the sensor 244 is mounted in a limiting through hole between the first part 2454 and the second part 2455. For example, the first part 2454, the sensor 244, and the second part 2455 may be stuck by using a gel dispensing process, to form an integral mechanical part. The first part 2454 may include a first body 24541, a first bump 24542, a first groove 24543, a second bump 24544, and a second groove 24545. The first bump 24542, the first groove 24543, the second groove 24545, and the second bump 24544 are located on a side that is of the first body 24541 and that faces the second part 2455. The first bump 24542 and the second bump 24544 are fastened to the first body 24541. The first groove 24543 and the second groove 24545 may be formed by recessing the first body 24541 in a direction facing away from the second part 2455. The second part 2455 may include a second body 24551, a third groove 24552, a fourth groove 24553, a fifth groove 24554, and a third bump 24555. The third groove 24552, the fourth groove 24553, the fifth groove 24554, and the third bump 24555 are all located on a side that is of the second body 24551 and that faces the first part 2454. The third bump 24555 is fastened to the second body 24551. The third groove 24552, the fourth groove 24553, and the fifth groove 24554 may be formed by recessing the second body 24551 in a direction facing away from the first part 2454. The first bump 24542 is mounted in the third groove 24552, the second bump 24544 is mounted in the fifth groove 24554, and the third bump 24555 is mounted in the second groove 24545, so that the first part 2454 is snap-fitted into the second part 2455. The first groove 24543 and the fourth groove 24553 are disposed opposite to each other and communicate with each other, to jointly form a limiting through hole of the sensor 244.

In this embodiment, the sensor 244 is stuck between the first part 2454 and the second part 2455 of the sensor base 245, and a temperature of an assembly environment is moderate, to prevent an enzyme at the sensing layer 2444 of the sensor 244 from being deactivated due to temperature impact. No heat insulation layer needs to be disposed in the region that is of the sensor 244 and that is in contact with the sensor base 245, and assembly between the sensor 244 and the sensor base 245 is simpler, to help reduce production costs of the physiological sign detection apparatus 100.

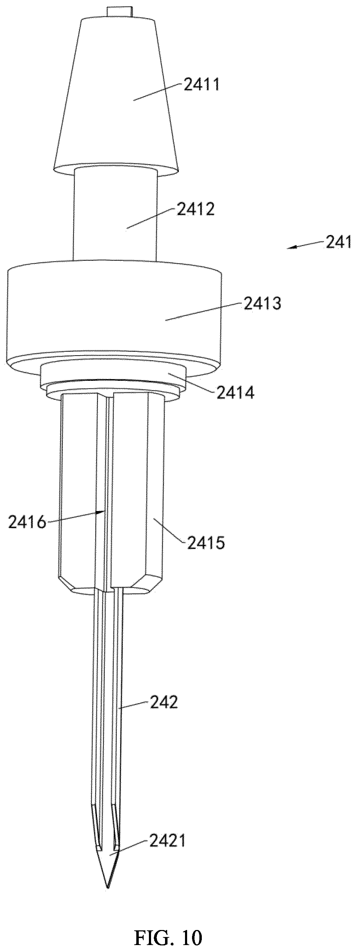

FIG. 10 is a diagram of an assembly structure of the puncture needle 242 and the needle base 241 that are shown in FIG. 6.

In some embodiments, the needle base 241 may include a limiting snap-fit 2411, a snap-fitting portion 2412, a limiting portion 2413, a sealing portion 2414, and a guiding portion 2415. The limiting snap-fit 2411, the snap-fitting portion 2412, the limiting portion 2413, the sealing portion 2414, and the guiding portion 2415 are sequentially connected. The limiting snap-fit 2411 may be generally in a shape of a circular truncated cone, the snap-fitting portion 2412 and the limiting portion 2413 may be generally in a shape of a cylinder, and a diameter of the snap-fitting portion 2412 is less than a diameter of a bottom surface of the limiting snap-fit 2411 and a diameter of the limiting portion 2413. The guiding portion 2415 has a recessed groove 2416, and the recessed groove 2416 extends in an axial direction of the needle base 241.

The puncture needle 242 is fastened to the needle base 241, and extends in the axial direction of the needle base 241. A puncture tip 2421 of the puncture needle 242 protrudes from the guiding portion 2415 of the needle base 241. A part that is of the puncture needle 242 and that is fastened to the guiding portion 2415 wraps the recessed groove 2416, and a shape of the recessed groove 2416 matches a shape of the puncture needle 242. The puncture needle 242 and the needle base 241 may be integrally formed. For example, the needle base 241 and the puncture needle 242 may be integrally formed by using the in-mold injection molding process. The needle base 241 may be made of engineering plastic such as polycarbonate or acrylonitrile butadiene styrene. The puncture needle 242 may be made of a metal material such as 316 stainless steel, 304 stainless steel, or medical-grade stainless steel (for example, SUS316L).

A cross section of the puncture needle 242 may be generally of a U-shaped structure, and the puncture needle 242 is configured to puncture and push away tissue of the organism 30. The implantation portion 2443 of the sensor 244 is located on an inner side of the puncture needle 242. The puncture tip 2421 of the puncture needle 242 may be configured to wrap the implantation portion 2443 of the sensor 244. When the puncture needle 242 is punctured into the organism 30, implantation space may be formed, so that the implantation portion 2443 of the sensor 244 is punctured into the organism 30. In addition, the puncture tip 2421 of the puncture needle 242 may further partially wrap the implantation portion 2443 of the sensor 244, and the implantation portion 2443 of the sensor 244 may also be punctured into the organism 30 with the puncture needle 242. This is not strictly limited in this application.

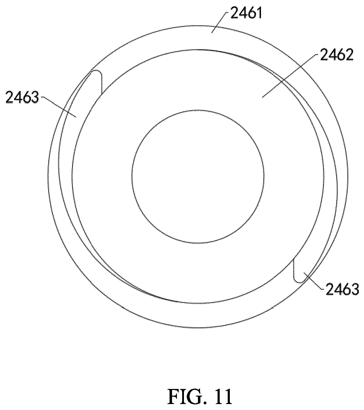

Refer to FIG. 6 and FIG. 11 together. FIG. 11 is a diagram of a structure of the sealing bottle 246 shown in FIG. 6 from another perspective.

In some embodiments, the sealing bottle 246 may include an abutting portion 2461, an accommodation portion 2462, and a ratchet 2463, the accommodation portion 2462 is connected to the abutting portion 2461, and the ratchet 2463 is convexly disposed on an outer side surface of the sealing bottle 246. There may be one or more ratchets 2463, and a plurality of ratchets 2463 are spaced apart around an axis of the sealing bottle 246. For example, the ratchet 2463 is fastened to an outer side surface of the accommodation portion 2462, and there is a smooth transition between an outer side surface of the ratchet 2463 and the outer side surface of the accommodation portion 2462. There may be two ratchets 2463, and the two ratchets 2463 are disposed opposite to each other.

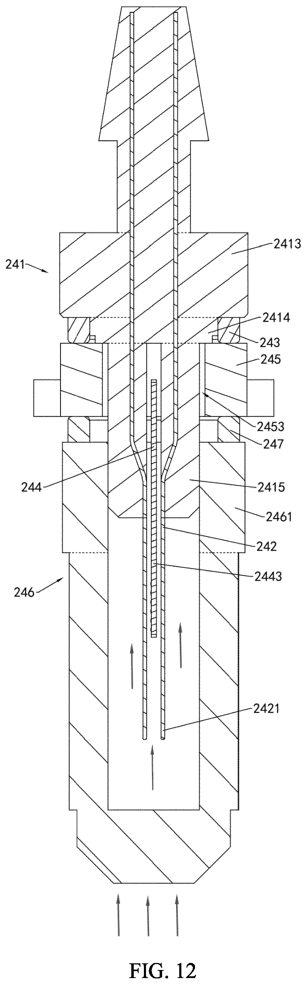

FIG. 12 is a diagram of a cross-sectional structure after the sensor sealing component 24 shown in FIG. 5 is cut along B-B.

In some embodiments, the sensor base 245 is located between the needle base 241 and the sealing bottle 246, an end of the needle base 241 passes through the sensor base 245, the sealing bottle 246 is detachably fastened to the needle base 241 and is hermetically connected to the sensor base 245, and the needle base 241 is hermetically connected to the sensor base 245. The guiding portion 2415 of the needle base 241 passes through the sensor base 245. For example, the guiding portion 2415 is inserted into the mounting through hole 2453 of the sensor base 245. The guiding portion 2415 is detachably fastened to the sealing bottle 246. For example, the sealing bottle 246 is threadedly connected to, snap-fitted into, or interference-connected to the needle base 241.

The needle base 241 may be threadedly connected to the sealing bottle 246. An outer side of the needle base 241 is provided with an external thread, an inner side of the sealing bottle 246 is provided with an internal thread (not shown in FIG. 12), and the internal thread of the sealing bottle 246 is engaged with the external thread of the needle base 241, so that the needle base 241 is threadedly connected to the sealing bottle 246. For example, an outer side of the guiding portion 2415 is provided with an external thread, an inner side of the abutting portion 2461 is provided with an internal thread, and the guiding portion 2415 of the needle base 241 is threadedly connected to the abutting portion 2461 of the sealing bottle 246. The needle base 241 is threadedly connected to the sealing bottle 246, so that the needle base 241 and the sealing bottle 246 are more stably connected, to prevent the sealing bottle 246 from being detached from the needle base 241 during transportation or when a collision occurs, thereby helping ensure stability of the physiological sign detection apparatus.

The first sealing member 243 is sleeved on an outer side of the sealing portion 2414 of the needle base 241, and is located between the limiting portion 2413 and the sensor base 245. The first sealing member 243 is hermetically connected to the sealing portion 2414 of the needle base 241 and the sensor base 245. The second sealing member 247 is located between the sealing bottle 246 and the sensor base 245, and is hermetically connected to the sealing bottle 246 and the sensor base 245. In this case, the puncture needle 242 wraps the implantation portion 2443 of the sensor 244. The sealing bottle 246 has a hollow cavity, and the implantation portion 2443 of the sensor 244 and the puncture tip 2421 of the puncture needle 242 are located in the hollow cavity of the sealing bottle 246.

In an existing sensor component, a sensor is exposed and is directly assembled with another component of the sensor component. In an assembly process, the sensor is easily touched, to damage a sensing material of an implantation portion of the sensor. Consequently, detection performance of the sensor is reduced, and detection accuracy is reduced. In this embodiment, the needle base 241 is threadedly connected to the sealing bottle 246, to securely connect the needle base 241, the first sealing member 243, the sensor base 245, the second sealing member 247, and the sealing bottle 246, so that the first sealing member 243 is hermetically connected to the needle base 241 and the sensor base 245, the second sealing member 247 is hermetically connected to the sensor base 245 and the sealing bottle 246, and the implantation portion 2443 of the sensor 244 is located in sealed space formed by using the sensor sealing component 24. In addition, the implantation portion 2443 of the sensor 244 is located in the hollow cavity of the sealing bottle 246, and the sealing bottle 246 may isolate and protect the implantation portion 2443 of the sensor 244, to avoid damage to the sensing layer 2444 of the implantation portion 2443 in a subsequent assembly process of the sensor 244 and another component, thereby helping improve reliability and detection accuracy of the physiological sign detection apparatus 100, and ensuring consistency of batches of sensors 244 in a production process.

The first sealing member 243 may be made of an elastic polymer material such as nitrile butadiene rubber, silicon rubber, or fluorine rubber. In some embodiments, the needle base 241 and the first sealing member 243 may be integrally formed by using a process such as a vulcanization process or a dual-shot injection molding process, so that the first sealing member 243 is formed on the sealing portion 2414 of the needle base 241. The needle base 241 and the first sealing member 243 are integrally formed, so that the first sealing member 243 and the needle base 241 are connected more stably, to help improve sealing performance between the needle base 241 and the first sealing member 243. In addition, when the needle base 241 is detached from the sensor base 245, the needle base 241 may be detached from the sensor base 245 together with the first sealing member 243. In some other embodiments, the first sealing member 243 may alternatively be fastened to the needle base 241 through bonding, or the like.

The second sealing member 247 may be made of an elastic polymer material such as nitrile butadiene rubber, silicon rubber, or fluorine rubber. In some embodiments, the second sealing member 247 and the sealing bottle 246 may be integrally formed by using a process such as a vulcanization process or a dual-shot injection molding process, so that the second sealing member 247 is fastened to a side that is of the sealing bottle 246 and that is close to the sensor base 245. The sealing bottle 246 and the second sealing member 247 are integrally formed, so that the second sealing member 247 and the sealing bottle 246 are connected more stably, to help improve sealing performance between the sealing bottle 246 and the second sealing member 247. In addition, when the sealing bottle 246 is detached from the needle base 241, the second sealing member 247 may be detached from the sensor base 245 with the sealing bottle 246. In some other embodiments, the second sealing member 247 may alternatively be fastened to the sealing bottle 246 through bonding, or the like.

In some embodiments, the sealing bottle 246 is threadedly connected to the needle base 241, and a ratchet 2463 is convexly disposed on the outer side surface of the sealing bottle 246. When the ratchet 2463 is subject to an action force opposite to a rotation direction of the internal thread of the sealing bottle 246, the sealing bottle 246 is detached from the needle base 241. For example, the physiological sign detection apparatus 100 further includes a housing cap 2 and a housing 1 (as shown in FIG. 2). The housing cap 2 is detachably fastened to the housing 1. For example, the housing cap 2 is threadedly connected to the housing 1. The housing cap 2 is provided with an internal thread (not shown in FIG. 2), the housing 1 is provided with an external thread, and the internal thread of the housing cap 2 is engaged with the external thread of the housing 1. A rotation direction of an internal thread of the housing cap 2 is the same as a rotation direction of an internal thread of the sealing bottle 246. In addition, the housing cap 2 is further provided with a pawl, and the pawl of the housing cap and the ratchet 2463 of the sealing bottle 246 are arranged in coordination. In a process in which the housing cap 2 is screwed on the housing 1, the pawl is slidably connected to the ratchet 2463. In other words, in the process in which the housing cap 2 is screwed on the housing 1, the pawl does not push the ratchet 2463 to move, to avoid a case in which the sealing bottle 246 is loose relative to the needle base 241 when the housing cap 2 is assembled to the housing 1. In a process in which the housing cap 2 is detached from the housing 1, the pawl is snap-fitted into the ratchet 2463. A rotation direction of the housing cap 2 when the housing cap 2 is detached from the housing 1 is opposite to a rotation direction of the internal thread of the sealing bottle 246, so that the housing cap 2 can simultaneously push the sealing bottle 246 to rotate. Because the sealing bottle 246 is threadedly connected to the needle base 241, the sealing bottle 246 can be screwed off relative to the needle base 241, so that the sealing bottle 246 is detached from the needle base 241. A quantity of circles of internal threads of the housing cap 2 may be greater than a quantity of circles of internal threads of the sealing bottle 246, so that the sealing bottle 246 may be detached from the needle base 241 before the housing cap 2 is detached from the housing 1.

In this embodiment, the housing cap 2 is threadedly connected to the housing 1, the sealing bottle 246 is threadedly connected to the needle base 241, and the pawl of the housing cap 2 cooperates with the ratchet 2463 of the sealing bottle 246. When the housing cap 2 is detached from the housing 1, the sealing bottle 246 may be pushed to be detached from the needle base 241, so that the puncture needle 242 and the sensor 244 are exposed, to help simplify operation steps of the physiological sign detection apparatus 100, and improve use experience of the user.

In some other embodiments, a connection between the needle base 241 and the sealing bottle 246 may also be a snap-fit connection, an interference-connection, or the like. This is not strictly limited in this application.

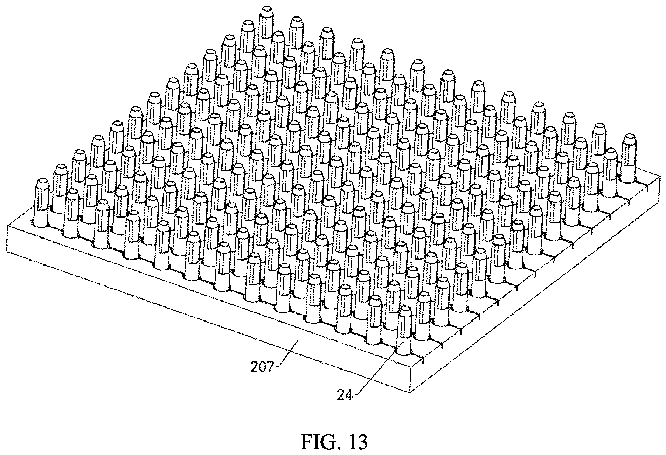

Refer to FIG. 12 and FIG. 13 together. FIG. 13 is a diagram of a structure of a sterilization tooling structure of the sensor sealing component 24 shown in FIG. 5.

This application provides a sterilization solution, to perform irradiation sterilization on puncture tips 2421 of puncture needles 242 and sensors 244 in batch sensor sealing assemblies 24 through ionizing radiation. In some embodiments, at least a part of the sealing bottle 246 allows ionizing radiation to pass through. For example, a part or all of regions of the sealing bottle 246 may allow ionizing radiation to pass through. The sealing bottle 246 may be made of engineering plastic such as polymethyl methacrylate (Polymethyl methacrylate, PMMA) and polycarbonate, and the engineering plastic such as methyl methacrylate and polycarbonate can both allow ionizing radiation to pass through.

Ionizing radiation may be one or a combination of electron beam radiation, gamma-ray radiation, and X-ray radiation. Ionizing radiation (as shown by an arrow in FIG. 12) passes through the sealing bottle 246, to sterilize the puncture tip 2421 of the puncture needle 242 and the implantation portion 2443 of the sensor 244. When irradiation sterilization is performed on the sensor sealing component 24, a plurality of sensor sealing assemblies 24 are placed in a positioning structure of a tray 207, the positioning structure of the tray 207 may be arranged in an array, a plurality of sensor sealing assemblies 24 may be placed on one tray 207, and the tray 207 is placed in a sterilization room for irradiation sterilization.

In the conventional technology, when radiation sterilization is performed on an assembled sensor component or physiological sign detection apparatus, a dedicated sterilization apparatus needs to be customized to implement fixed-point sterilization on a puncture tip of a puncture needle and an implantation portion of a sensor. Fixed-point sterilization has a high requirement on a radiation device, and is difficult to be implemented in batched. In another manner in the conventional technology, when irradiation sterilization is performed on the entire sensor component or the entire physiological sign detection apparatus, a specific tooling needs to be additionally designed, and the sensor component or the physiological sign detection apparatus is assembled with the specific tooling, so that the specific tooling performs shielding protection on an internal circuit of the sensor component, and then irradiation sterilization can be performed. Consequently, sterilization procedures are cumbersome and inefficient.

In this embodiment, at least the partial region of the sealing bottle 246 allows ionizing radiation to pass through, so that the sensor sealing component 24 can sterilize the puncture tip 2421 of the puncture needle 242 and the implantation portion 2443 of the sensor 244 through irradiation. The sensor sealing component 24 of the sensor component 20 provides a sealed environment for the implantation portion 2443 of the sensor 244 and the puncture tip 2421 of the puncture needle 242, so that in a sterilization process, the sensor sealing component 24 may be sterilized independently, and then the sterilized sensor sealing component 24 is assembled with another structure such as an electronic part of the sensor component 20. Therefore, no additional protective fixture is needed when performing radiation sterilization on the sensor sealing component 24, making sterilization procedures simpler and more efficient. In addition, after sterilization, as a sterile barrier, the sealing bottle 246 may form good isolation protection for the puncture tip 2421 of the puncture needle 242 and the implantation portion 2443 of the sensor 244. The puncture tip 2421 of the puncture needle 242 and the implantation portion 2443 of the sensor 244 are not contaminated for a second time. In subsequent packaging, a sterilized package for ensuring that the sensor 244 is located in a sterile environment does not need to be additionally added to a physiological sign detection apparatus 100, to reduce costs. In addition, the sensor sealing component 24 has a small volume, and a plurality of sensor sealing assemblies 24 can be sterilized simultaneously through one radiation sterilization procedure, to greatly improve sterilization efficiency and reducing sterilization costs.

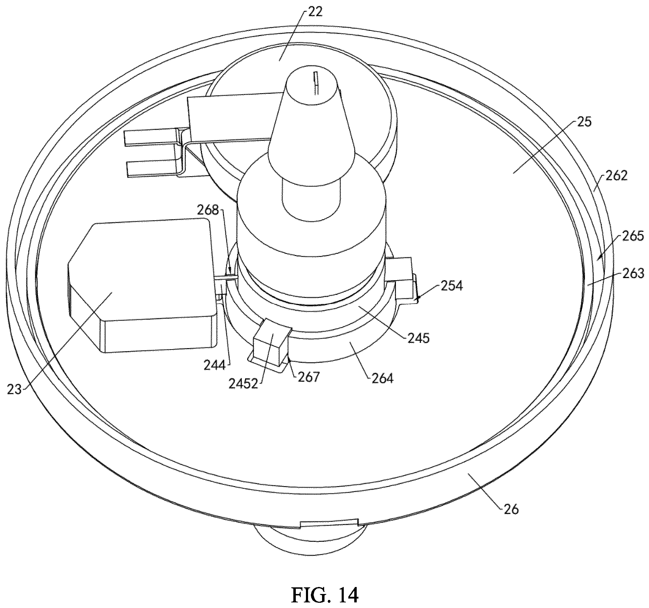

Refer to FIG. 5 and FIG. 14 together. FIG. 14 is a diagram of a partial structure of the sensor component 20 shown in FIG. 4.

In some embodiments, the lower cover 26 may include a bottom plate 261, a first side plate 262, a second side plate 263, and a blocking flange 264. The bottom plate 261 of the lower cover 26 may be generally in a disc shape. The first side plate 262 of the lower cover 26 surrounds and is connected to a circumferential edge of the bottom plate 261 of the lower cover 26, the second side plate 263 is located on an inner side of the first side plate 262 and is located on a same side of the bottom plate 261 of the lower cover 26 as the first side plate 262, a circumferential edge of the second side plate 263 is fastened to the bottom plate 261 of the lower cover 26, and a mounting groove 265 is formed between the first side plate 262 and the second side plate 263.

The lower cover 26 is provided with a first through hole 266, the first through hole 266 is located on an inner side of the second side plate 263 and runs through the bottom plate 261 of the lower cover 26, the blocking flange 264 is disposed around the first through hole 266, and the blocking flange 264 is located on a same side of the bottom plate 261 of the lower cover 26 as the first side plate 262. A side that is of the blocking flange 264 and that faces away from the bottom plate 261 of the lower cover 26 has a first avoidance groove 267. There may be one or more first avoidance grooves 267, and a plurality of first avoidance grooves 267 are spaced apart in a circumferential direction of the blocking flange 264. For example, there may be three first avoidance grooves 267. The side that is of the blocking flange 264 and that faces away from the bottom plate 261 of the lower cover 26 is further provided with a blocking groove 268, and the blocking groove 268 and the first avoidance groove 267 are spaced apart.

In some embodiments, the circuit board 25 has first mounting space 251, second mounting space 252, and a third through hole 253. The first mounting space 251 and the second mounting space 252 are spaced apart, and the third through hole 253 communicates with the first mounting space 251 and is spaced apart from the second mounting space 252. The first mounting space 251 and the second mounting space 252 may be through holes, or may be open grooves, and an opening of the open groove is disposed facing away from the bottom plate 261 of the lower cover 26. The circuit board 25 has a second avoidance groove 254, there are one or more second avoidance grooves 254, and a plurality of second avoidance grooves 254 are located on a circumferential side of the third through hole 253 and communicate with the third through hole 253. For example, there may be three second avoidance grooves 254.

The circuit board 25 is fastened to the bottom plate 261 of the lower cover 26, and is located on an inner side of the second side plate 263. The blocking flange 264 is inserted into the third through hole 253, the first mounting space 251 is disposed opposite to the blocking groove 268 in the radial direction, and the plurality of second avoidance grooves 254 are disposed opposite to the plurality of first avoidance grooves 267 in a one-to-one correspondence in the radial direction. The circuit board 25 and the lower cover 26 may form an integral mechanical part by using an adhesive process. For example, a contact surface between the bottom plate 261 of the lower cover 26 and the circuit board 25 may be coated with glue, so that the circuit board 25 is stuck and fastened to the lower cover 26.

The sensor sealing component 24 runs through the first through hole 266 and the second through hole 215, and is snap-fitted into the lower cover 26 and/or the upper cover 21. For example, the sensor sealing component 24 is inserted into the first through hole 266 of the lower cover 26, and the blocking block 2452 of the sensor base 245 is snap-fitted into the first avoidance groove 267, so that the sensor base 245 is snap-fitted into the lower cover 26. The sensor 244 passes through the blocking groove 268 and is connected to the connector 23. The connector 23 is fastened to the circuit board 25 and is electrically connected to the circuit board 25. The sensor 244 is electrically connected to the connector 23, to be electrically connected to the circuit board 25 by using the connector 23. The battery 22 is fastened to the circuit board 25 and is electrically connected to the circuit board 25, to supply power to the sensor component 20. There may be one or more batteries 22. For example, a single battery 22 or two batteries 22 connected in series may be used as a power supply of the sensor component 20, and a voltage of the battery 22 may be 1.5 volts (volt, V).

In this embodiment, the circuit board 25 and the lower cover 26 form an integral mechanical part by using an adhesive process, so that the circuit board 25 and a bottom plate 261 of the lower cover 26 are stably fastened, to avoid a collision caused by movement of the circuit board 25 relative to the lower cover 26 in a transportation process of the physiological sign detection apparatus 100, and help improve reliability of the physiological sign detection apparatus 100. The first mounting space 251 and the second avoidance groove 254 on the circuit board 25 may be used for positioning and/or limiting, so that the circuit board 25 is aligned with and assembled with the lower cover 26. In addition, the second avoidance groove 254 of the circuit board 25 may further avoid the blocking block 2452 of the sensor base 245, to prevent the blocking block 2452 from abutting against the circuit board 25 and cause a failure of the circuit board 25.

In some other embodiments, the circuit board 25 may also be fastened to a side that is of the upper cover 21 and that faces the lower cover 26. This is not strictly limited in this application.

In some embodiments, a center of the first through hole 266 of the lower cover 26 is located or approximately located on an axis of the sensor component 20, and the sensor sealing component 24 is snap-fitted into the blocking flange 264, so that the puncture needle 242 and the implantation portion 2443 of the sensor 244 are located or close to the axis of the sensor component 20, and the puncture needle 242 and the implantation portion 2443 of the sensor 244 can extend in the middle of the lower cover 26. Based on the foregoing structural design, when the puncture needle 242 and the implantation portion 2443 of the sensor 244 are punctured into the organism 30, the puncture needle 242 and the implantation portion 2443 of the sensor 244 are subject to an even action force, and the puncture needle 242 is not easily skewed. This helps ensure that the puncture needle 242 is quickly punctured into the organism 30, and the organism 30 feels a small pain feeling.

In some other embodiments, the blocking flange 264 may alternatively be located on the upper cover 21, and the sensor sealing component 24 is snap-fitted into the blocking flange 264, so that the sensor sealing component 24 is snap-fitted into the upper cover 21. This is not strictly limited in this application.

In addition, in the sensor component 20, the sensor sealing component 24 is arranged in the middle, the circuit board 25 is arranged around the sensor sealing component 24, the battery 22, the connector 23, and another component are arranged on a circumferential side of the sensor sealing component 24, and a plurality of components of the sensor component 20 are arranged compactly, to facilitate miniaturization.

In some embodiments, a diameter of the sensor component 20 may be less than 21 millimeters (mm), and a thickness is less than 3 millimeters. For example, a diameter of the sensor component 20 may be 20.6 millimeters, 20 millimeters, 19.7 millimeters, 19.5 millimeters, 18.8 millimeters, 18.3 millimeters, or 18 millimeters. The thickness of the sensor component 20 may be 2.95 millimeters, 2.9 millimeters, 2.85 millimeters, 2.8 millimeters, 2.78 millimeters, or the like. The sensor component 20 has a small volume, to help reduce a volume of the implanter 10 configured to implant the sensor component 20.

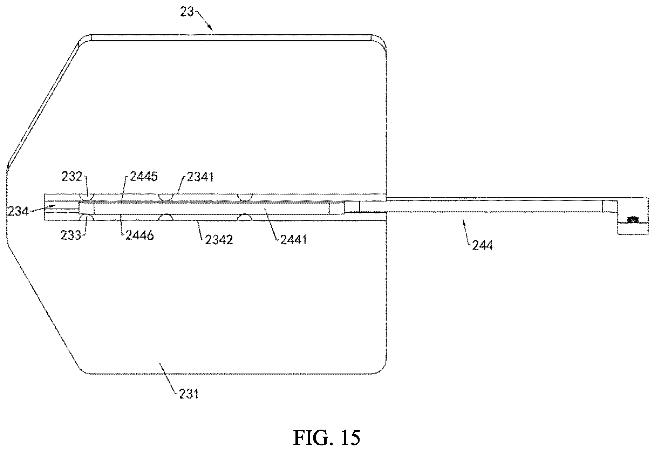

FIG. 15 is a diagram of an assembly structure of a sensor 244 and a connector 23 that are shown in FIG. 14.

In some embodiments, the connector 23 may include a connector body 231, a plurality of first spring plates 232, and a plurality of second spring plates 233. The connector body 231 is provided with a connecting groove 234, and the connecting groove 234 may include a first groove wall 2341 and a second groove wall 2342 that are disposed opposite to each other. The plurality of first spring plates 232 and the plurality of second spring plates 233 are both fastened to the connector body 231, at least a part of the plurality of first spring plates 232 is convexly disposed on the first groove wall 2341, at least a part of the plurality of second spring plates 233 is convexly disposed on the second groove wall 2342, and a gap is formed between the plurality of first spring plates 232 and the plurality of second spring plates 233. The connection portion 2441 of the sensor 244 is snap-fitted into the connecting groove 234 of the connector 23, and is electrically connected to the plurality of first spring plates 232 and the plurality of second spring plates 233. For example, the sensor 244 may be generally plate-like, the sensor 244 may include a first surface 2445 and a second surface 2446 that are disposed facing away from each other, and the first surface 2445 and the second surface 2446 each are provided with a conductive layer. The plurality of first spring plates 232 of the sensor 244 are electrically connected to a conductive layer of the first surface 2445, and the plurality of second spring plates 233 of the sensor 244 are electrically connected to a conductive layer of the second surface 2446.

In the conventional technology, when a sensor is electrically connected to a circuit board, a connection portion of the sensor needs to be folded, so that the connection portion of the sensor is parallel to the circuit board, to be welded with a pad of the circuit board. Consequently, costs of the sensor are increased, and a folding position of the sensor is easily damaged. In addition, only one surface of the sensor can be welded to the circuit board, and a large connection area needs to be set for each of the sensor and the circuit board. Consequently, a volume of the sensor and a volume of the circuit board are large. In this embodiment, at least a part of the plurality of first spring plates 232 of the connector 23 is convexly disposed on the first groove wall 2341 of the connecting groove 234, and at least a part of the plurality of second spring plates 233 is convexly disposed on the second groove wall 2342 of the connecting groove 234. A conductive layer on a surface of the sensor 244 can be electrically connected to the plurality of first spring plates 232 and the plurality of second spring plates 233 only by snap-fitting the sensor 244 into the connecting groove 234 of the connector 23. Assembly between the sensor 244 and the connector 23 is simpler, and an electrical connection between the sensor 244 and the connector 23 is more stable. In addition, the sensor 244 can maintain a plate-like structure, and does not need to be folded additionally due to an electrical connection requirement, so that the sensor 244 has high reliability. In addition, because spring plates are convexly disposed on groove walls on two sides of the connecting groove 234, the connector 23 has more contacts configured to be electrically connected to the sensor 244, to reduce an arrangement area of the first spring plate 232 and the second spring plate 233, thereby reducing a volume of the connector 23, helping miniaturize the sensor component 20. In addition, miniaturization of the sensor component 20 also helps miniaturize the physiological sign detection apparatus 100.

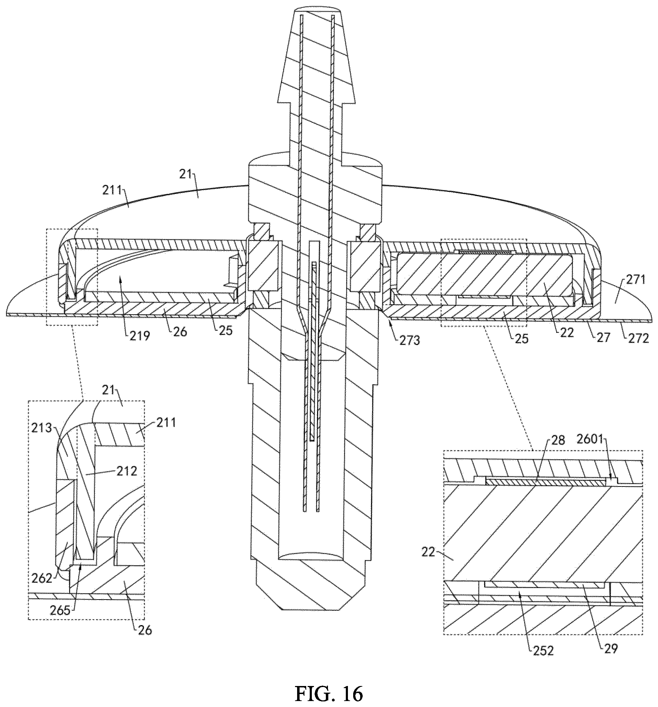

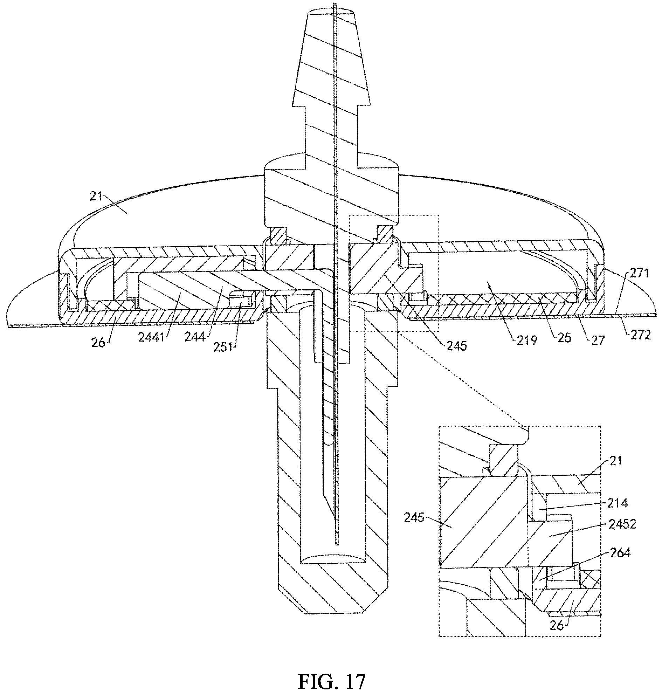

Refer to FIG. 5, FIG. 16, and FIG. 17 together. FIG. 16 is a diagram of a cross-sectional structure after the sensor component 20 shown in FIG. 4 is cut along C-C in some embodiments. FIG. 17 is a diagram of a cross-sectional structure after the sensor component 20 shown in FIG. 4 is cut along D-D in some embodiments.

In some embodiments, the upper cover 21 is fastened to the lower cover 26, accommodation space 219 is formed between the upper cover 21 and the lower cover 26, and the circuit board 25, the battery 22, the connector 23, the connection portion 2441 of the sensor 244, and at least a part of the sensor base 245 are located in the accommodation space 219. The upper cover 21 may include a top plate 211, a side plate 212, a side plate flange 213, and a snap-fitting flange 214, the side plate 212 of the upper cover 21 surrounds and is connected to a circumferential edge of the top plate 211 of the upper cover 21, and the side plate flange 213 surrounds and is connected to an outer circumferential side of the side plate 212 of the upper cover 21. The side plate 212 of the upper cover 21 is mounted in the mounting groove 265 of the lower cover 26, so that the upper cover 21 is fastened to the lower cover 26. The side plate flange 213 abuts against the first side plate 262 of the lower cover 26. The upper cover 21 and the lower cover 26 may form an integral mechanical part by using an adhesive process. For example, all contact surfaces between the lower cover 26 and the upper cover 21 are coated with glue, so that the lower cover 26 and the upper cover 21 are stuck and fastened.

In this embodiment, the mounting groove 265 of the lower cover 26 has positioning and limiting roles, so that the upper cover 21 is mounted and fastened to the lower cover 26. In addition, the side plate flange 213 of the upper cover 21 abuts against the first side plate 262 of the lower cover 26, and the upper cover 21 and the lower cover 26 may be integrated by using an adhesive, so that sealing performance between the upper cover 21 and the lower cover 26 is better, to avoid a case in which a contaminant enters the accommodation space 219 from a gap between the upper cover 21 and the lower cover 26, and consequently, an electronic element 204 located in the accommodation space 219 is damaged.

The connection portion 2441 of the sensor 244 is mounted in the first mounting space 251 of the circuit board 25. Components of the sensor 244 may further include a positive spring plate 28 and a negative spring plate 29. A side that is of the top plate 211 of the upper cover 21 and that faces the lower cover 26 has an accommodation groove 2601, an end of the positive spring plate 28 is mounted in the accommodation groove 2601 and is in contact with and electrically connected to the battery 22, and an end of the negative spring plate 29 is mounted in the second mounting space 252 of the circuit board 25, and is in contact with and electrically connected to the battery 22. The other end of the positive spring plate 28 and the other end of the negative spring plate 29 may be fastened and electrically connected to the circuit board 25, to conduct the battery 22 and the circuit board 25.

The upper cover 21 has a second through hole 215, the second through hole 215 is located on the top plate 211 of the upper cover 21, and the second through hole 215 directly faces and communicates with the first through hole 266. The sensor base 245 is snap-fitted into the lower cover 26 and/or the upper cover 21. To be specific, the sensor base 245 is snap-fitted into the lower cover 26, or the sensor base 245 is snap-fitted into the lower cover 26 and the upper cover 21, or the sensor base 245 is snap-fitted into the upper cover 21. For example, after the circuit board 25, the sensor sealing component 24, the connector 23, and the battery 22 are all first mounted on the lower cover 26, the needle base 241 passes through the second through hole 215 of the upper cover 21, and then the upper cover 21 is mounted on the lower cover 26. The blocking block 2452 of the sensor base 245 is located between the blocking flange 264 of the lower cover 26 and the snap-fitting flange 214 of the upper cover 21, and the blocking block 2452 is snap-fitted into the snap-fitting flange 214 and/or the blocking flange 264, so that the sensor base 245 is snap-fitted into the lower cover 26 and/or the upper cover 21. For example, there are three blocking blocks 2452, to ensure that the sensor base 245 is stably snap-fitted into the lower cover 26 and/or the upper cover 21. The sensor base 245 is exposed from the first through hole 266 and exposed from the second through hole 215. A part of the sensor base 245 may be located in the first through hole 266 and the second through hole 215.

In this embodiment, the sensor base 245 is snap-fitted into the lower cover 26 and/or the upper cover 21, so that the sensor base 245 is stably fastened to the lower cover 26 and the upper cover 21, to avoid a case in which the sensor 244 is loose relative to the lower cover 26 and/or the upper cover 21 when the sealing bottle 246 and the needle base 241 are detached from the sensor base 245. The lower cover 26 has the first through hole 266, and the sensor base 245 is exposed from the first through hole 266, so that the sealing bottle 246 can pass through the first through hole 266 and is exposed from the lower cover 26. The upper cover 21 has the second through hole 215, and the sensor base 245 is exposed from the second through hole 215, so that the needle base 241 can pass through the second through hole 215 of the upper cover 21. In this way, components of the sensor sealing component 24 are assembled, and then assembled with the lower cover 26 and the upper cover 21, to protect the implantation portion 2443 of the sensor 244 by the sealing bottle 246 and reduce a damage risk.

In some embodiments, the bonding member 27 has a first surface 271 and a second surface 272 that are disposed facing away from each other, the first surface 271 of the bonding member 27 is stuck on a side that is of the lower cover 26 and that faces away from the upper cover 21, and the second surface 272 of the bonding member 27 is configured to be stuck on the organism when the sensor component 20 abuts against the organism, so that the sensor component 20 is stuck on a skin surface of the organism. For example, the bonding member 27 may be generally plate-like, the bonding member 27 has a fourth through hole 273, the fourth through hole 273 is disposed opposite to the first through hole 266, and the sensor base 245 is exposed from the fourth through hole 273, so that the sealing bottle 246 can pass through the fourth through hole 273 and be hermetically connected to the sensor base 245. The bonding member 27 may be a medical back adhesive, and the bonding member 27 can maintain stable bonding for at least 14 days.

Refer to FIG. 2, FIG. 10, and FIG. 17. In some embodiments, the implanter 10 is provided with a hook (not shown in FIG. 2), and the hook of the implanter 10 is snap-fitted into the snap-fitting portion 2412 of the needle base 241. The implanter 10 is configured to push the sensor component 20 to move toward a position at which the organism 30 is located, so that the bonding member 27 of the sensor component 20 can be stuck on the organism. After the sensor 244 is implanted into the organism 30 under guidance of the puncture needle 242, the hook of the implanter 10 drives, by using the needle base 241, the puncture needle 242 to move in a direction away from the organism 30, so that the puncture needle 242 is detached from the organism.

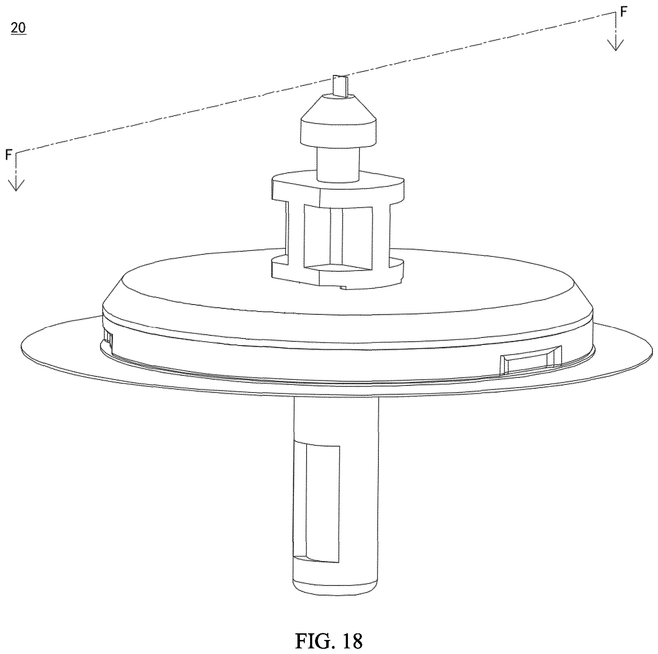

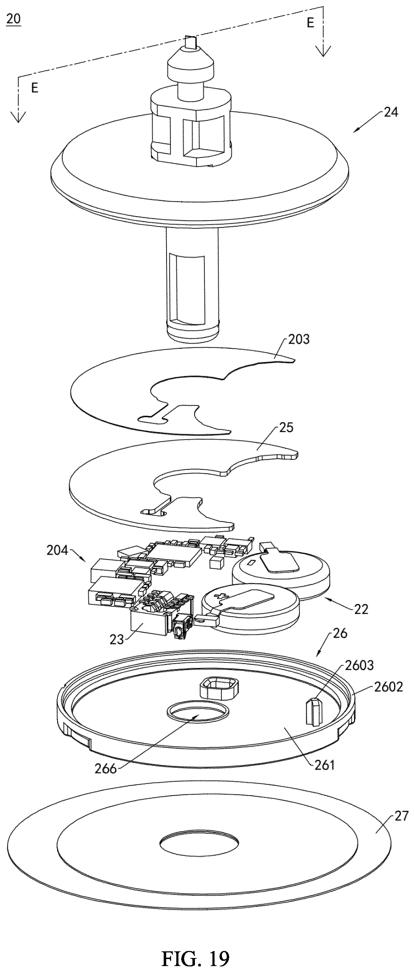

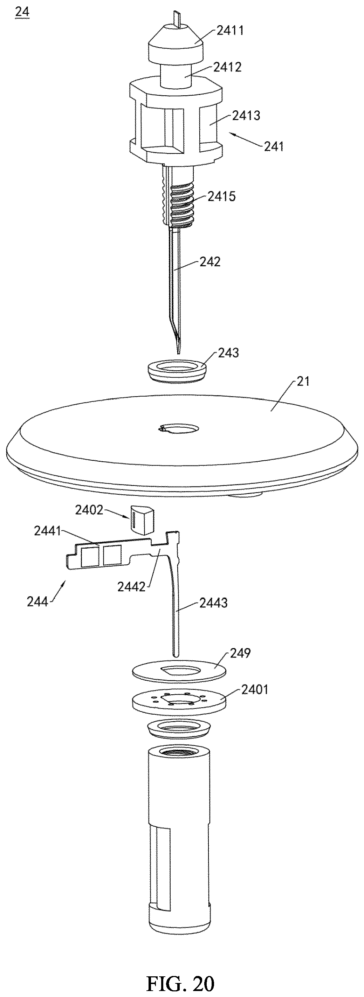

FIG. 18 is a diagram of a structure of the sensor component 20 shown in FIG. 2 in some other embodiments. FIG. 19 is a diagram of a partial exploded structure of the sensor component 20 shown in FIG. 18. FIG. 20 is a diagram of a partial exploded structure of the sensor sealing component 24 shown in FIG. 19. This embodiment may include most of the technical solutions in the foregoing embodiments. The following mainly describes differences between this embodiment and the foregoing embodiments, and most same content of this embodiment and the foregoing embodiments is not described again.

In some embodiments, the sensor component 20 may include a sensor sealing component 24, a first bonding member 203, a circuit board 25, a battery 22, a connector 23, an electronic element 204, a lower cover 26, and a bonding member 27. The sensor sealing component 24 may include a needle base 241, a puncture needle 242, a first sealing member 243, an upper cover 21, a sensor 244, a fastener 2402, a second bonding member 249, a gasket 2401, a second sealing member 247, and a sealing bottle 246. Both the upper cover 21 and the lower cover 26 may be made of engineering plastic such as polycarbonate or acrylonitrile butadiene styrene. A chip, an NFC antenna, and the like may be disposed on the circuit board 25. The electronic element 204 may include a capacitor, a resistor, Bluetooth, and the like. The second bonding member 249 and the gasket 2401 each have a through hole, and the through hole of the gasket 2401 directly faces the through hole of the second bonding member 249. The puncture needle 242, most structures of the needle base 241, and the bonding member 27 may be disposed with reference to the foregoing embodiments. Details are not described again in this application.

In some embodiments, the needle base 241 may include a limiting snap-fit 2411, a snap-fitting portion 2412, a limiting portion 2413, and a guiding portion 2415. The limiting snap-fit 2411, the snap-fitting portion 2412, the limiting portion 2413, and the guiding portion 2415 are sequentially connected. The limiting snap-fit 2411 may be generally in a shape of a circular truncated cone, the snap-fitting portion 2412 may be generally in a shape of a cylinder, and a diameter of the snap-fitting portion 2412 is less than a diameter of the limiting snap-fit 2411 and a diameter of the limiting portion 2413. The snap-fitting portion 2412 of the needle base 241 is configured to be snap-fitted into the hook of the implanter. The sensor 244 may include a connection portion 2441, a fastening portion 2442, and an implantation portion 2443. The fastening portion 2442 is connected to the implantation portion 2443 and the connection portion 2441. The connection portion 2441 of the sensor 244 is configured to be electrically connected to the connector 23, the implantation portion 2443 of the sensor 244 is configured to be punctured into the organism, and the implantation portion 2443 is provided with a sensing layer 2444 (not shown in FIG. 20). A manufacturing process of the bonding member 27, the puncture needle 242, and the sensor 244, and assembly of the puncture needle 242, the sensor 244, and the needle base 241 may be set with reference to the foregoing embodiments. Details are not described again below.

In some embodiments, the lower cover 26 may include a bottom plate 261, a side plate 2602, and a positioning protrusion 2603. The side plate 2602 of the lower cover 26 surrounds and is connected to a circumferential edge of the bottom plate 261 of the lower cover 26. The lower cover 26 is provided with a first through hole 266. An end of the positioning protrusion 2603 is fastened to the bottom plate 261 of the lower cover 26, and both the positioning protrusion 2603 and the side plate 2602 of the lower cover 26 are located on a same side of the bottom plate 261 of the lower cover 26.

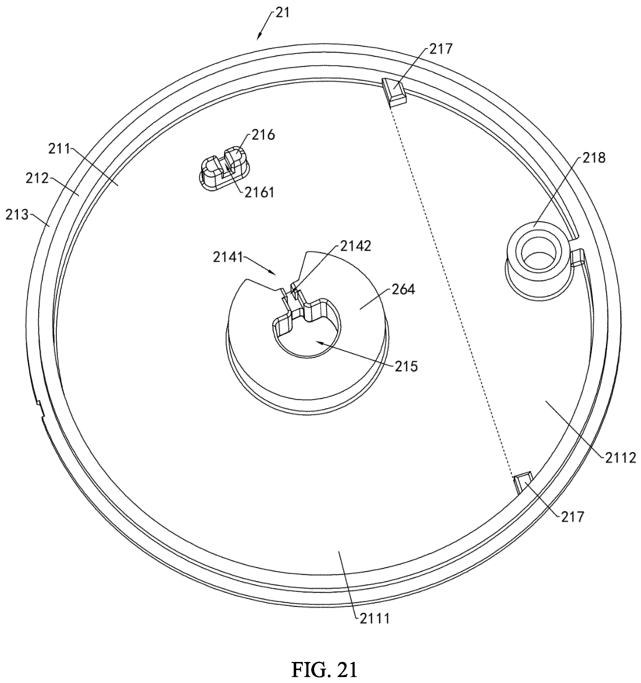

FIG. 21 is a diagram of a structure of the upper cover 21 shown in FIG. 20 from another perspective.

In some embodiments, the upper cover 21 may include a top plate 211, a side plate 212, and a side plate flange 213. The top plate 211 of the upper cover 21 may be generally in a disc shape. The side plate 212 of the upper cover 21 surrounds and is connected to a circumferential edge of the top plate 211 of the upper cover 21. The side plate flange 213 surrounds and is connected to an outer circumferential side of the side plate 212 of the upper cover 21. The upper cover 21 has a second through hole 215, the second through hole 215 is located on the top plate 211 of the upper cover 21, and a center of the second through hole 215 approximately coincides with a center of the top plate 211 of the upper cover 21.

The upper cover 21 may further include a blocking flange 264, the blocking flange 264 is disposed around the second through hole 215, and the blocking flange 264 and the side plate 212 of the upper cover 21 are located on a same side of the top plate 211 of the upper cover 21. The blocking flange 264 has accommodation space 2141 and a blocking groove 2142, and the blocking groove 2142 communicates with the accommodation space 2141 and the second through hole 215. For example, the accommodation space 2141 may be a notch. The upper cover 21 may further include a first positioning member 216, the first positioning member 216 is spaced apart from the blocking flange 264, and the first positioning member 216 is disposed opposite to the accommodation space 2141 of the blocking flange 264. The first positioning member 216 has a limiting groove 2161. An extension direction of the limiting groove 2161 is the same as an extension direction of the blocking groove 2142, and both the limiting groove 2161 and the blocking groove 2142 are located or approximately located in a same straight line.

The upper cover 21 may further include a second positioning member 217 and a positioning flange 218. The second positioning member 217 and the positioning flange 218 are both spaced apart from the first positioning member 216 and the blocking flange 264. The positioning flange 218 has an accommodation groove. For example, there are two second positioning members 217, and the two second positioning members 217 are opposite and spaced apart from each other.

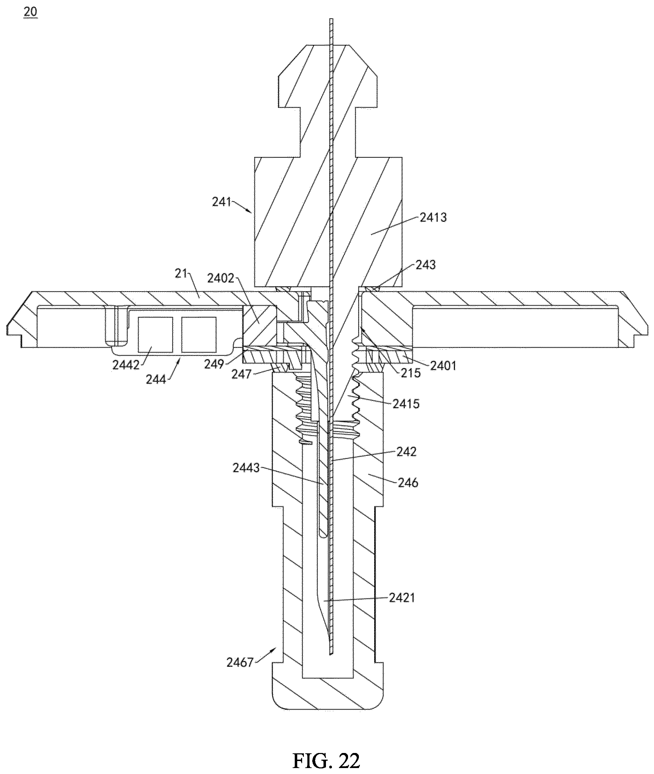

Refer to FIG. 21 and FIG. 22 together. FIG. 22 is a diagram of a cross-sectional structure after the sensor sealing component 24 shown in FIG. 19 is cut along E-E.

In some embodiments, the sensor 244 is snap-fitted into the upper cover 21, the upper cover 21 is located between the needle base 241 and the sealing bottle 246, an end of the needle base 241 passes through the upper cover 21, the sealing bottle 246 is detachably fastened to the needle base 241, and is hermetically connected to the upper cover 21, and the needle base 241 is hermetically connected to the upper cover 21. The guiding portion 2415 of the needle base 241 is inserted into the second through hole 215 of the upper cover 21, and the guiding portion 2415 is detachably fastened to the sealing bottle 246. For example, the needle base 241 may be threadedly connected to the sealing bottle 246, the outer side of the guiding portion 2415 is provided with the external thread, the inner side of the sealing bottle 246 is provided with the internal thread, and the guiding portion 2415 is threadedly connected to the sealing bottle 246. In some other embodiments, a connection between the needle base 241 and the sealing bottle 246 may also be a snap-fit connection, an interference-connection, or the like. This is not strictly limited in this application.

The first sealing member 243 is mounted between the limiting portion 2413 of the needle base 241 and the upper cover 21, and the first sealing member 243 is hermetically connected to the limiting portion 2413 of the needle base 241 and the upper cover 21. The fastening portion 2442 of the sensor 244 is snap-fitted into the blocking groove 2142 of the upper cover 21, and a tail end of the connection portion 2441 is snap-fitted into the limiting groove 2161 of the upper cover 21. The blocking flange 264 and the first positioning member 216 position and limit the sensor 244, and the sensor 244 can slightly move in the blocking groove 2142 and the limiting groove 2161, to prevent the sensor 244 from being stuck or worn out. The fastener 2402 fills in the accommodation space 2141 of the blocking flange 264, and is configured to fasten the fastening portion 2442 of the sensor 244 with the upper cover 21. The implantation portion 2443 of the sensor 244 is located on an inner side of the puncture needle 242. The second bonding member 249 may be a double-sided adhesive, a side of the second bonding member 249 is stuck on the blocking flange 264, and the other side of the second bonding member 249 is stuck on the gasket 2401, to fasten the gasket 2401 to the blocking flange 264. The second sealing member 247 is located between the gasket 2401 and the sealing bottle 246, and is hermetically connected to the sealing bottle 246 and the gasket 2401. In this case, the puncture needle 242 wraps the implantation portion 2443 of the sensor 244. The sealing bottle 246 has a hollow cavity, and the implantation portion 2443 of the sensor 244 and the puncture tip 2421 of the puncture needle 242 are located in the hollow cavity of the sealing bottle 246.

In an existing sensor component, a sensor is exposed and is directly assembled with another component of the sensor component. In an assembly process, the sensor is easily touched, to damage a sensing material of an implantation portion of the sensor. Consequently, detection performance of the sensor is reduced, and detection accuracy is reduced. In this embodiment, the needle base 241 is connected to the sealing bottle 246 threadedly or in another manner, to securely connect the needle base 241, the first sealing member 243, the upper cover 21, the fastener 2402, the gasket 2401, the second sealing member 247, and the sealing bottle 246, so that the first sealing member 243 is hermetically connected to the needle base 241 and the sensor base 245, the gasket 2401 and the second sealing member 247 are hermetically connected to the sensor base 245 and the sealing bottle 246, and the implantation portion 2443 of the sensor 244 is located in sealed space formed by using the sensor sealing component 24. In addition, the implantation portion 2443 of the sensor 244 is located in the hollow cavity of the sealing bottle 246, and the sealing bottle 246 may isolate and protect the implantation portion 2443 of the sensor 244, to avoid damage to the sensing layer 2444 of the implantation portion 2443 in a subsequent assembly process of the sensor 244 and another component, thereby helping improve reliability and detection accuracy of the physiological sign detection apparatus 100, and ensuring consistency of batches of sensors 244 in a production process.

The first sealing member 243 may be made of an elastic polymer material such as nitrile butadiene rubber, silicon rubber, or fluorine rubber. In some embodiments, the needle base 241 and the first sealing member 243 may be integrally formed by using a process such as a vulcanization process or a dual-shot injection molding process, so that the first sealing member 243 is formed on the limiting portion 2413 of the needle base 241. The needle base 241 and the first sealing member 243 are integrally formed, so that the first sealing member 243 and the needle base 241 are connected more stably, to help improve sealing performance between the needle base 241 and the first sealing member 243. In addition, when the needle base 241 is detached from the upper cover 21, the needle base 241 may be detached from the upper cover 21 together with the first sealing member 243. In some other embodiments, the first sealing member 243 may alternatively be fastened to the needle base 241 through bonding, or the like. This is not strictly limited in this application.

The second sealing member 247 may be made of an elastic polymer material such as nitrile butadiene rubber, silicon rubber, or fluorine rubber. In some embodiments, the second sealing member 247 and the sealing bottle 246 may be integrally formed by using a process such as a vulcanization process or a dual-shot injection molding process, so that the second sealing member 247 is fastened to a side that is of the sealing bottle 246 and that is close to the sensor base 245. The sealing bottle 246 and the second sealing member 247 are integrally formed, so that the second sealing member 247 and the sealing bottle 246 are connected more stably, to help improve sealing performance between the sealing bottle 246 and the second sealing member 247. In addition, when the sealing bottle 246 is detached from the needle base 241, the second sealing member 247 may be detached from the gasket 2401 with the sealing bottle 246. In some other embodiments, the second sealing member 247 may alternatively be fastened to the sealing bottle 246 through bonding, or the like.

In some embodiments, the outer side surface of the sealing bottle 246 is provided with a snap-fitting groove 2467, the housing cap 2 (as shown in FIG. 2) is provided with the pawl, and the snap-fitting groove 2467 is configured to cooperate with the pawl of the housing cap 2, so that the housing cap 2 can drive the sealing bottle 246 to rotate. In addition, the ratchet described above may also be disposed on the outer side surface of the sealing bottle 246. In some other embodiments, the sealing bottle 246 may be snap-fitted into or interference-connected to the needle base 241, and an end that is of the sealing bottle 246 and that faces away from the needle base 241 is provided with a boss.

In some embodiments, the fastener 2402 may be formed by curing glue, so that while the sensor 244 and the upper cover 21 are fastened, shape adaptation is better implemented, and difficulty in molding and assembling is low. For example, the fastener 2402 may be ultraviolet cured glue, and the glue can be quickly cured by irradiating ultraviolet light or visible light, thereby improving assembly efficiency. The sealing bottle 246 may be made of an opaque material, for example, a black engineering plastic such as polymethyl methacrylate and polycarbonate. In an ultraviolet light curing process of the fastener 2402, ultraviolet light does not radiate to the implantation portion 2443 of the sensor 244 through the sealing bottle 246, so that an enzyme at the sensing layer 2444 (not shown in FIG. 22) can be prevented from being deactivated due to impact of the ultraviolet light, thereby helping improve detection accuracy of the physiological sign detection apparatus 100. In some other embodiments, the fastener 2402 may also be formed by curing another glue, for example, may be normal-temperature cured glue.

In some other embodiments, the sensor sealing component 24 may not be provided with the second bonding member 249 and the gasket 2401, and the sealing bottle 246 may be directly hermetically connected to the upper cover 21 and the fastener 2402 by using the second sealing member 247. This is not strictly limited in this application.

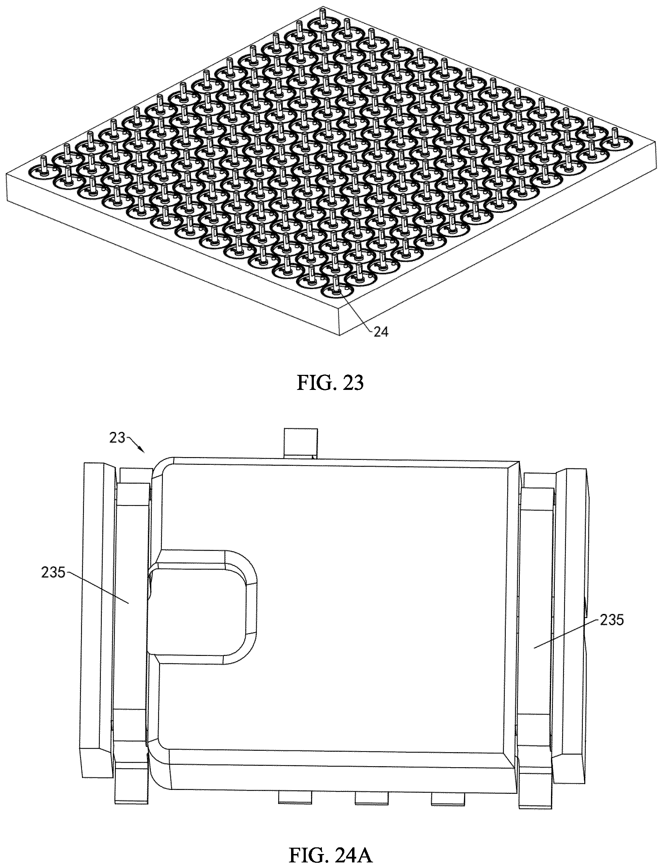

Refer to FIG. 23 and FIG. 22 together. FIG. 23 is a diagram of a structure of a sterilization tooling structure of the sensor sealing component 24 shown in FIG. 19.

This application provides a sterilization solution. A volume of the sensor sealing component 24 is small, and a plurality of sensor sealing assemblies 24 may be placed in a positioning structure of a same tray, to perform irradiation sterilization on puncture tips 2421 of puncture needles 242 and sensors 244 in batch sensor sealing assemblies 24 through ionizing radiation. Radiation sterilization of the sensor sealing component 24 may be set with reference to the foregoing embodiments. Details are not described again in this application.

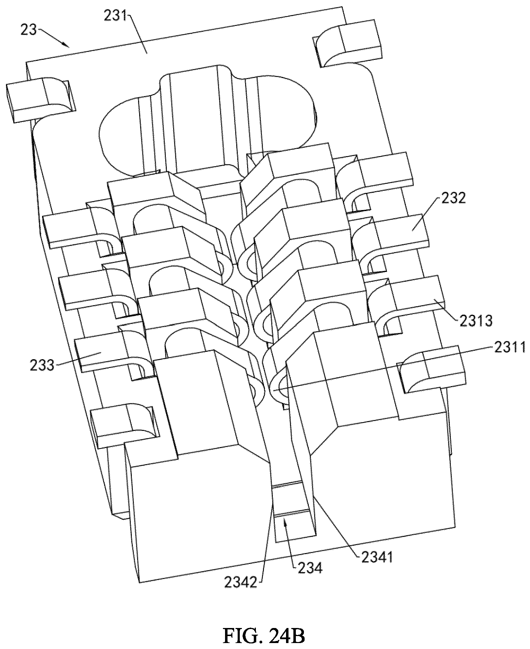

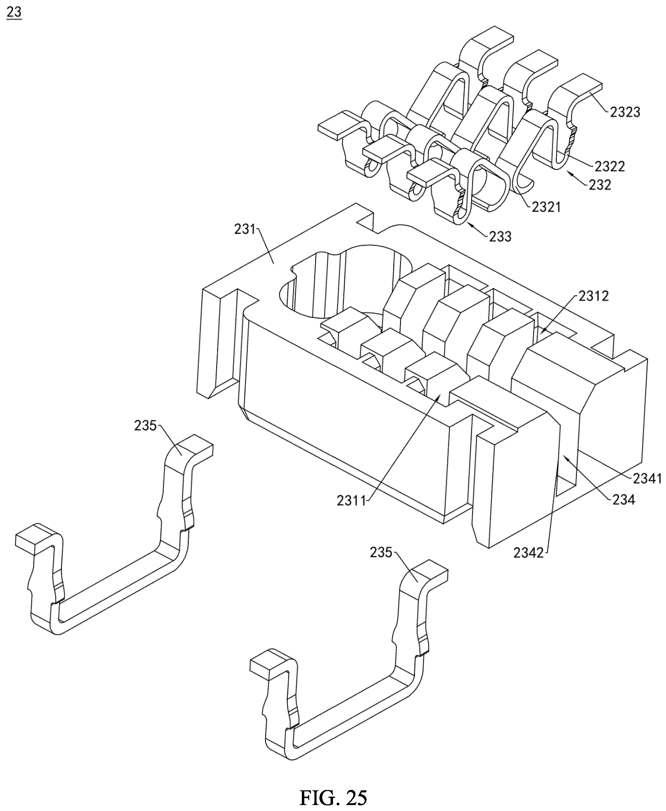

FIG. 24A is a diagram of a structure of the connector 23 shown in FIG. 19 in some embodiments. FIG. 24B is a diagram of a structure of the connector 23 shown in FIG. 24A from another perspective. FIG. 25 is a diagram of an exploded structure of the connector 23 shown in FIG. 24B.

In some embodiments, the connector 23 may include a connector body 231, a plurality of first spring plates 232, and a plurality of second spring plates 233. The plurality of first spring plates 232 and the plurality of second spring plates 233 are disposed opposite to each other in a one-to-one correspondence, and the plurality of first spring plates 232 and the plurality of second spring plates 233 are all fastened to the connector body 231. The connector body 231 has a connecting groove 234, and the connecting groove 234 includes a first groove wall 2341 and a second groove wall 2342 that are disposed opposite to each other. At least a part of the plurality of first spring plates 232 is convexly disposed on the first groove wall 2341, at least a part of the plurality of second spring plates 233 is convexly disposed on the second groove wall 2342, and a gap is formed between the plurality of first spring plates 232 and the plurality of second spring plates 233. The connector body 231 further has a first mounting groove 2311 and a second mounting groove 2312, and the first mounting groove 2311 and the second mounting groove 2312 are disposed opposite to each other. The first mounting groove 2311 and the second mounting groove 2312 are respectively located on two sides of the connecting groove 234, and both are connected to the connecting groove 234. The first spring plate 232 is mounted in the first mounting groove 2311, and the second spring plate 233 is mounted in the second mounting groove 2312. For example, there may be a plurality of first mounting grooves 2311 and a plurality of second mounting grooves 2312, and a quantity of first mounting grooves 2311 and a quantity of second mounting grooves 2312 are respectively commensurate with a quantity of first spring plates 232 and a quantity of second spring plates 233.

The first spring plate 232 may include an abutting portion 2321, a fastening portion 2322, and a connection portion 2323. The fastening portion 2322 is connected to the abutting portion 2321 and the connection portion 2323. The abutting portion 2321 is an elastic flexible end, and may move in a direction close to or away from the fastening portion 2322. The fastening portion 2322 is mounted in the first mounting groove 2311 and is fastened to the connector body 231. The connection portion 2323 protrudes from the connector body 231. The abutting portion 2321 of the first spring plate 232 protrudes from the first groove wall 2341 of the connecting groove 234. The second spring plate 233 may include an abutting portion, a fastening portion, and a connection portion. The abutting portion, the fastening portion, and the connection portion of the second spring plate 233 are disposed with reference to the first spring plate 232. Details are not described again below.

For example, the first spring plate 232 and the second spring plate 233 each may be an integral mechanical part formed by bending a metal plate. The first spring plate 232 and the second spring plate 233 each include two plate surfaces disposed opposite to each other and a side surface connecting the two plate surfaces. A distance between two plate surfaces is a thickness of a spring plate, and the side surface is a surface on which the thickness of the spring plate is located. Surfaces that are of the first spring plate 232 and the second spring plate 233 and that are disposed opposite to each other in the connecting groove 234 are plate surfaces. In other words, a plate surface of the first spring plate 232 and a plate surface of the second spring plate 233 are disposed opposite to each other in the connecting groove 234.

In some embodiments, the connector 23 further includes a reinforcing member 235, and the reinforcing member 235 is fastened to the connector body 231. There may be two reinforcing members 235, and the two reinforcing members 235 are respectively fastened to two ends of the connector body 231.

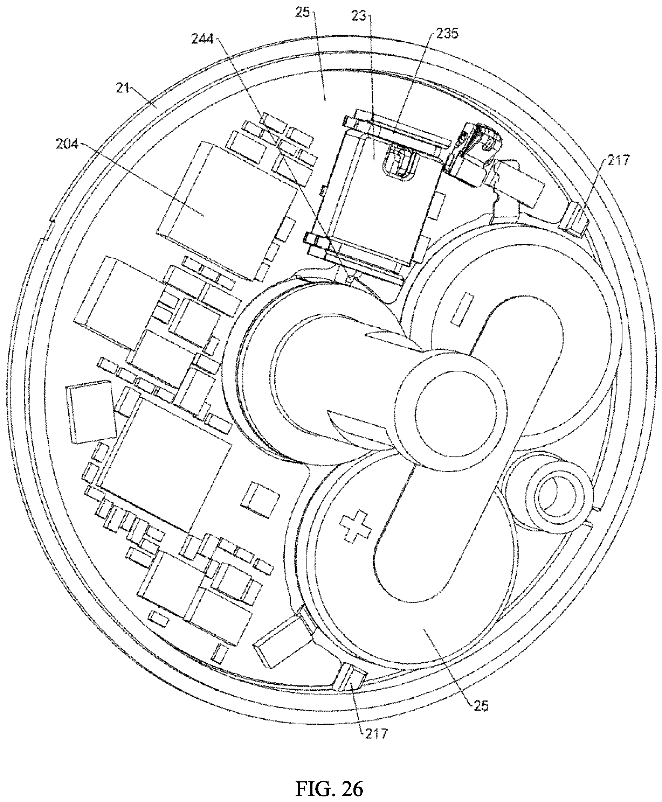

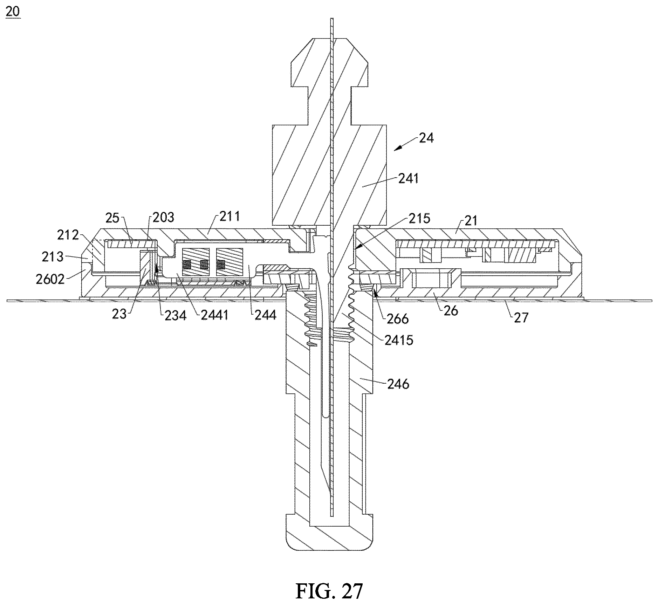

Refer to FIG. 21, FIG. 26, and FIG. 27 together. FIG. 26 is a diagram of the partial structure of the sensor component 20 shown in FIG. 18. FIG. 27 is a diagram of a cross-sectional structure after the sensor component 20 shown in FIG. 18 is cut along F-F.

In some embodiments, the top plate 211 of the upper cover 21 may include a first region 2111 and a second region 2112, the circuit board 25 is fastened in the first region 2111, and the two second positioning members 217 disposed opposite to each other position and limit the circuit board 25. The first bonding member 203 may be a double-sided adhesive, a side of the first bonding member 203 is stuck on the top plate 211 of the upper cover 21, and the other side of the first bonding member 203 is stuck on the circuit board 25. The battery 22 is fastened to the second region 2112, and is electrically connected to the circuit board 25. The electronic element 204 is fastened to the circuit board 25. The connector 23 is fastened and electrically connected to the circuit board 25. Both the connection portion 2323 of the first spring plate 232 and the connection portion of the second spring plate 233 may be welded to the circuit board 25, and the reinforcing member 235 may be welded to the circuit board 25. The connection portion 2441 of the sensor 244 is snap-fitted into the connecting groove 234 of the connector 23, and is electrically connected to the plurality of first spring plates 232 and the plurality of second spring plates 233 (not shown in FIG. 27). For example, the sensor 244 is mounted between abutting portions 2321 of the plurality of first spring plates 232 and abutting portions of the plurality of second spring plates 233, and is electrically connected to the abutting portions 2321 of the plurality of first spring plates 232 and the abutting portions of the plurality of second spring plates 233.

In this embodiment, the sensor 244 is electrically connected to the abutting portions 2321 of the plurality of first spring plates 232 and the abutting portions of the plurality of second spring plates 233. A plate surface of the first spring plate 232 is in contact with the sensor 244, and a plate surface of the second spring plate 233 is in contact with the sensor 244. The sensor 244 is in face contact with the first spring plate 232 and the second spring plate 233, a contact area is large, the sensor 244 is subject to an even force, and the sensor 244 is not easily damaged. In addition, when a tail end of the connection portion 2441 of the sensor 244 is snap-fitted into a first positioning member 216, the tail end may move slightly in a limiting groove 2161. Therefore, the connection portion 2441 of the sensor 244 is electrically connected to an abutting portion 2321 of the first spring plate 232 and an abutting portion 23 of the second spring plate 233, and a position of the connection portion 2441 of the sensor 244 may be slightly adjusted to implement self-adaptation, to better abut against the first spring plate 232 and the second spring plate 233, so that a connecting position is subject to an even force, thereby ensuring that the sensor 244 is stably connected to the first spring plate 232 and the second spring plate 233, to reduce fatigue caused by an uneven force applied to the first spring plate 232 and the second spring plate 233 for a long period of time.

In some embodiments, after the sensor sealing component 24 is completely assembled, both the circuit board 25 and the battery 22 are fastened to the top plate 211 of the upper cover 21, and then assembly of the electronic elements 204 and the connector 23 is completed. Finally, the sealing bottle 246 passes through the first through hole 266 of the lower cover 26, and the lower cover 26 is fastened to the upper cover 21, so that assembly of the sensor component 20 can be completed. The side plate 2602 of the lower cover 26 is snap-fitted into the side plate 212 and the side plate flange 213 of the upper cover 21, the positioning protrusion 2603 (as shown in FIG. 19) is mounted in the accommodation groove of the positioning flange 218, and the positioning protrusion 2603 cooperates with the positioning flange 218, so that the upper cover 21 and the lower cover 26 are assembled and fastened.

In this embodiment, the sensor sealing component 24 includes the upper cover 21, and the upper cover 21 only needs to be provided with the second through hole 215 that allows the guiding portion 2415 of the needle base 241 to pass through. A hole diameter of the second through hole 215 is smaller. In addition, the needle base 241 is fastened to the upper cover 21 by being securely connected to the sealing bottle 246. Assembly between the upper cover 21 and the needle base 241 is simpler, and the lower cover 26 does not need to be provided with a structure for being snap-fitted into the sensor sealing component 24, to help simplify a structure of the lower cover 26.

Refer to FIG. 2, FIG. 20, and FIG. 27. In some embodiments, the implanter 10 is provided with a hook (not shown in FIG. 2), and the hook of the implanter 10 is snap-fitted into the snap-fitting portion 2412 of the needle base 241. The implanter 10 is configured to push the sensor component 20 to move toward a position at which the organism 30 is located, so that the bonding member 27 of the sensor component 20 can be stuck on the organism. After the sensor 244 is implanted into the organism 30 under guidance of the puncture needle 242, the hook of the implanter 10 drives, by using the needle base 241, the puncture needle 242 to move in a direction away from the organism 30, so that the puncture needle 242 is detached from the organism.

It may be understood that the sensor component 20 in the foregoing other embodiments may include the sensor sealing component 24 in this embodiment. In addition, other implementation structures of the sensor component 20 including the sensor sealing component 24 in this embodiment may also be of a plurality of types. The sensor component 20 may be used in various types of implanters.

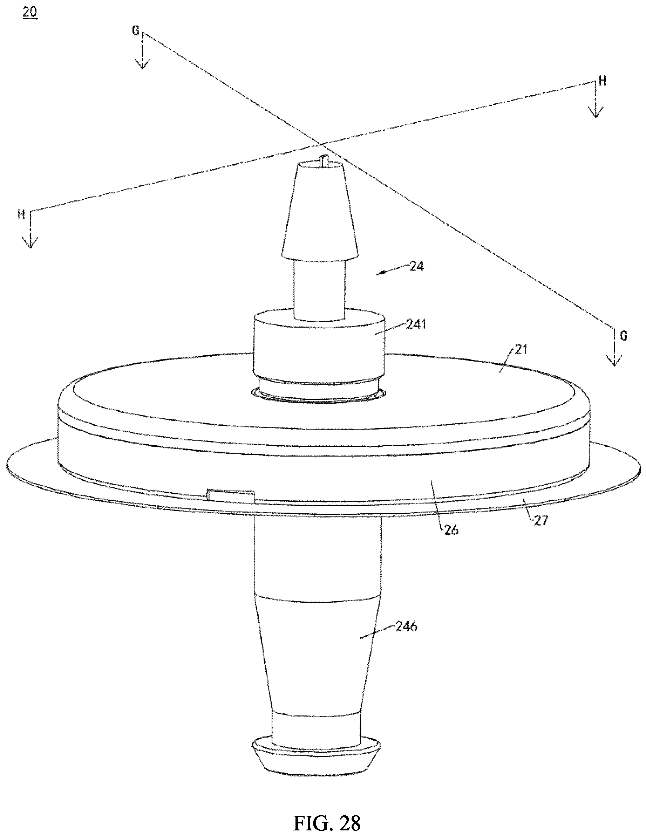

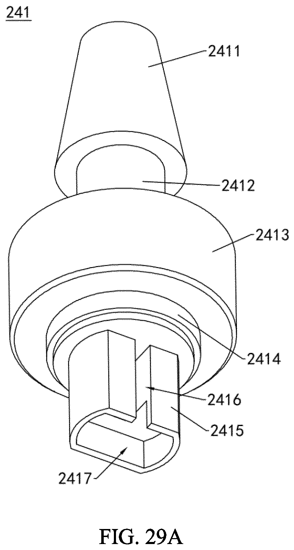

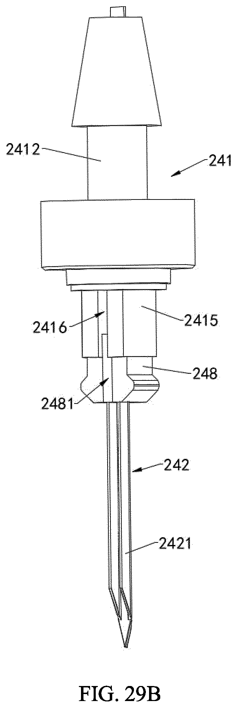

FIG. 28 is a diagram of a structure of the sensor component 20 shown in FIG. 2 in some other embodiments. FIG. 29A is a diagram of a structure of a needle base 241 of the sensor component 20 shown in FIG. 28. FIG. 29B is a diagram of an assembly structure of a snap-fit 248 and the needle base 241 in some embodiments.

In some embodiments, the sensor component 20 may include an upper cover 21, a sensor sealing component 24, a battery, a connector, a circuit board, a lower cover 26, and a bonding member 27. The sensor sealing component 24 may include a needle base 241, a puncture needle 242, a first sealing member, a sensor, a sensor base, a second sealing member, and a sealing bottle 246. The battery, the connector, the circuit board, the sensor, the sensor base, and the second sealing member are not shown in FIG. 28 to FIG. 29B. In this embodiment, the upper cover 21, the battery, the connector, the circuit board, the lower cover 26, the bonding member 27, the first sealing member, the sensor, and the sensor base of the sensor component 20 may be disposed with reference to the foregoing embodiments. Details are not described again below. A main difference between this embodiment and the foregoing embodiments lies in that the needle base 241 and a sealing bottle 246 are different.

In some embodiments, the needle base 241 may include a limiting snap-fit 2411, a snap-fitting portion 2412, a limiting portion 2413, a sealing portion 2414, and a guiding portion 2415. The limiting snap-fit 2411, the snap-fitting portion 2412, the limiting portion 2413, the sealing portion 2414, and the guiding portion 2415 are sequentially connected. The limiting snap-fit 2411 may be generally in a shape of a circular truncated cone, the snap-fitting portion 2412 and the limiting portion 2413 may be generally in a shape of a cylinder, and a diameter of the snap-fitting portion 2412 is less than a diameter of the limiting snap-fit 2411 and a diameter of the limiting portion 2413. The guiding portion 2415 has a recessed groove 2416, and the recessed groove 2416 extends in an axial direction of the needle base 241. The guiding portion 2415 further has a positioning groove 2417, and an opening of the positioning groove 2417 faces a side that faces away from the sealing portion 2414. The sensor sealing component 24 may further include a snap-fit 248, an end of the snap-fit 248 is mounted in the positioning groove 2417 of the guiding portion 2415, and a flange is disposed at the other end that is of the snap-fit 248 and that faces away from the guiding portion 2415. A side surface of the snap-fit 248 has a recessed groove 2481, and the recessed groove 2481 of the snap-fit 248 is connected to the recessed groove 2416 of the guiding portion 2415.

The puncture needle 242 is fastened to the needle base 241 and the snap-fit 248, and extends in an axial direction of the needle base 241. The puncture tip 2421 of the puncture needle 242 protrudes from the snap-fit 248. A part that is of the puncture needle 242 and that is fastened to the guiding portion 2415 and the snap-fit 248 wraps the recessed groove 2416 of the guiding portion 2415 and the recessed groove 2481 of the snap-fit 248, and both shapes of the recessed groove 2416 of the guiding portion 2415 and the recessed groove 2481 of the snap-fit 248 match a shape of the puncture needle 242.

The needle base 241, the snap-fit 248, and the puncture needle 242 may be integrally formed. For example, the needle base 241 and the snap-fit 248 may be integrally formed by using a vulcanization process or a dual-shot injection molding process. The needle base 241 and the puncture needle 242 may be integrally formed by using the in-mold injection molding process. The needle base 241 may be made of engineering plastic such as polycarbonate or acrylonitrile butadiene styrene. The snap-fit 248 may be made of an elastic polymer material such as rubber, polyurethane, or thermoplastic polyurethane elastomer rubber (Thermoplastic polyurethanes, TPU). The puncture needle 242 may be made of a metal material such as 316 stainless steel, 304 stainless steel, or medical-grade stainless steel (for example, SUS316L).

A cross section of the puncture needle 242 may be generally of a U-shaped structure, and the puncture needle 242 is configured to puncture and push away tissue of the organism 30. An implantation portion (not shown in FIG. 29B) of the sensor may be located on an inner side of the puncture needle 242. The puncture tip 2421 of the puncture needle 242 wraps the implantation portion of the sensor. When the puncture needle 242 is punctured into the organism 30, implantation space may be formed, so that the implantation portion of the sensor is punctured into the organism 30. In addition, the puncture tip 2421 of the puncture needle 242 may further partially wrap the implantation portion of the sensor, and the implantation portion of the sensor may also be punctured into the organism 30 with the puncture needle 242. This is not strictly limited in this application.

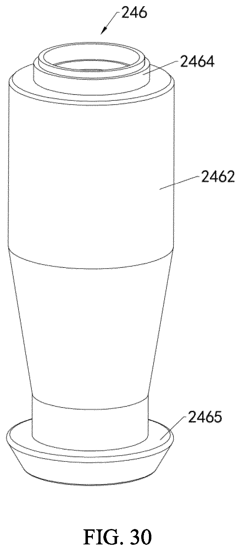

FIG. 30 is a diagram of a structure of the sealing bottle 246 shown in FIG. 28 from another perspective.

In some embodiments, the sealing bottle 246 may include a sleeving portion 2464, an accommodation portion 2462, and a boss 2465, the accommodation portion 2462 is connected to the sleeving portion 2464 and the boss 2465, and the boss 2465 is located at an end that is of the sealing bottle 246 and that faces away from the needle base 241. A diameter of the sleeving portion 2464 is less than a diameter of the accommodation portion 2462.

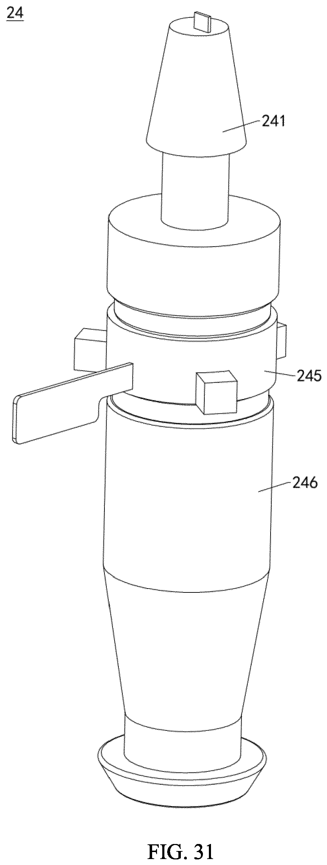

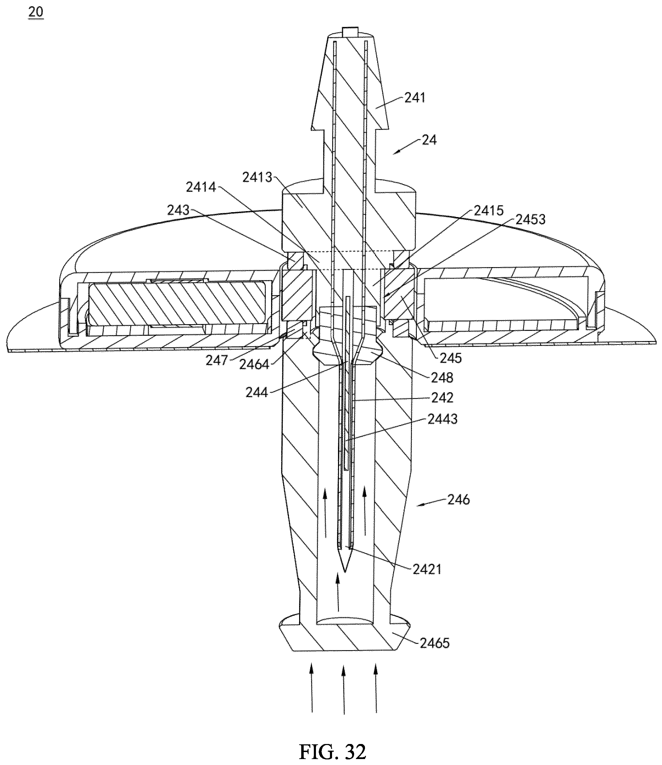

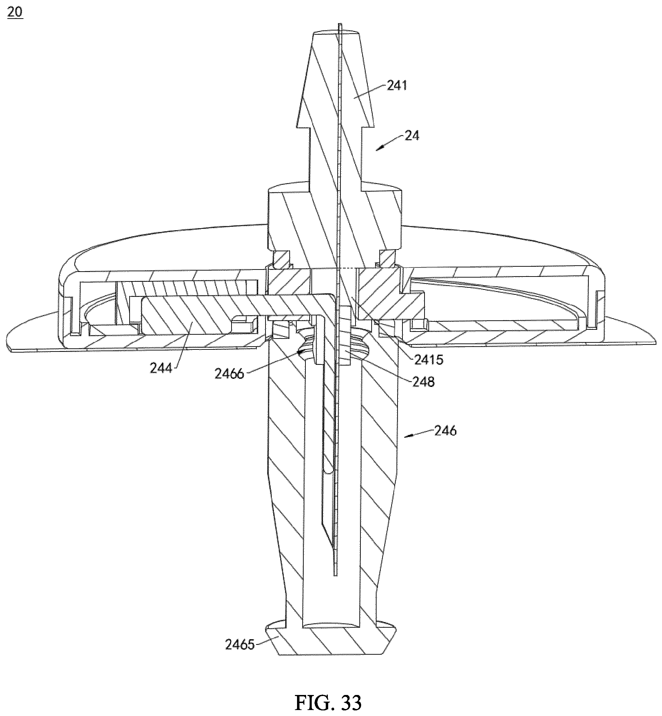

FIG. 31 is a diagram of a structure of the sensor sealing component 24 shown in FIG. 28. FIG. 32 is a diagram of a cross-sectional structure after the sensor component 20 shown in FIG. 28 is cut along G-G. FIG. 33 is a diagram of a cross-sectional structure after the sensor component 20 shown in FIG. 28 is cut along H-H.

In some embodiments, the sensor base 245 is located between the needle base 241 and the sealing bottle 246, an end of the needle base 241 passes through the sensor base 245, the sealing bottle 246 is snap-fitted into or interference-connected to the needle base 241 and is hermetically connected to the sensor base 245, and the needle base 241 is hermetically connected to the sensor base 245. A snap-fitting groove 2466 is disposed on an inner side of the sealing bottle 246, and the snap-fitting groove 2466 is located at an end that is of the sealing bottle 246 and that faces away from the boss 2465. For example, a flange of the snap-fit 248 may be mounted in the snap-fitting groove 2466 of the sealing bottle 246, so that the snap-fit 248 is snap-fitted into or interference-connected to the sealing bottle 246. The guiding portion 2415 of the needle base 241 passes through the sensor base 245, and the snap-fit 248 is snap-fitted into or interference-connected to the sealing bottle 246. The guiding portion 2415 is inserted into the mounting through hole 2453 of the sensor base 245, and can move relative to the sensor base 245. The first sealing member 243 is sleeved on an outer side of the sealing portion 2414, and is located between the limiting portion 2413 and the sensor base 245. The first sealing member 243 is hermetically connected to the sealing portion 2414 of the needle base 241 and the sensor base 245. The second sealing member 247 is sleeved on the sleeving portion 2464 of the sealing bottle 246, and is located between the sealing bottle 246 and the sensor base 245. The second sealing member 247 is hermetically connected to the sealing bottle 246 and the sensor base 245. In this case, the puncture needle 242 wraps the implantation portion 2443 of the sensor 244. The sealing bottle 246 has a hollow cavity, and the implantation portion 2443 of the sensor 244 and the puncture tip 2421 of the puncture needle 242 are located in the hollow cavity of the sealing bottle 246.

In an existing sensor component, a sensor is exposed and is directly assembled with another component of the sensor component. In an assembly process, the sensor is easily touched, to damage a sensing material of an implantation portion of the sensor. Consequently, detection performance of the sensor is reduced, and detection accuracy is reduced. In this embodiment, the sealing bottle 246 is snap-fitted into or interference-connected to the needle base 241 by using the snap-fit 248, to securely connect the needle base 241, the first sealing member 243, the sensor base 245, the second sealing member 247, and the sealing bottle 246, so that the first sealing member 243 is hermetically connected to the needle base 241 and the sensor base 245, the second sealing member 247 is hermetically connected to the sensor base 245 and the sealing bottle 246, and the implantation portion 2443 of the sensor 244 is located in sealed space formed by using the sensor sealing component 24. In addition, the implantation portion 2443 of the sensor 244 is located in the hollow cavity of the sealing bottle 246, and the sealing bottle 246 may isolate and protect the implantation portion 2443 of the sensor 244, to avoid damage to the sensing layer 2444 of the implantation portion 2443 in a subsequent assembly process of the sensor 244 and another component, thereby helping improve reliability and detection accuracy of the physiological sign detection apparatus.

In some embodiments, when the boss 2465 is subject to an action force away from the needle base 241, the sealing bottle 246 is detached from the needle base 241. The housing cap 2 (as shown in FIG. 2) is removably fastened to the housing 1. For example, the housing cap 2 is provided with a snap-fitting jaw, and the snap-fitting jaw of the housing cap 2 is snap-fitted with the boss 2465 of the sealing bottle 246. The housing cap 2 is snap-fitted into or interference-connected to the housing 1. In a process in which the housing cap 2 is detached from the housing 1, the snap-fitting jaw is snap-fitted with the boss 2465, the snap-fitting jaw drives the sealing bottle 246 to move toward a side away from the sensor component 20, and the housing cap 2 drives the sealing bottle 246 to be detached from the needle base 241.

In this embodiment, the housing cap 2 is snap-fitted into or interference-connected to the housing 1, the sealing bottle 246 is snap-fitted into or interference-connected to the needle base 241, and the snap-fitting jaw of the housing cap 2 cooperates with the boss 2465 of the sealing bottle 246. When the housing cap 2 is detached from the housing 1, the sealing bottle 246 may be driven to be detached from the needle base 241, so that the puncture needle 242 is exposed, to help simplify operation steps of the physiological sign detection apparatus 100 and improve user experience.

The second sealing member 247 may be made of an elastic polymer material such as nitrile butadiene rubber, silicon rubber, or fluorine rubber. The second sealing member 247 and the sealing bottle 246 may be integrally formed by using a process such as a vulcanization process or a dual-shot injection molding process, so that the second sealing member 247 is fastened to a side that is of the sealing bottle 246 and that is close to the sensor base 245. The sealing bottle 246 and the second sealing member 247 are integrally formed, so that the second sealing member 247 and the sealing bottle 246 are connected more stably, to help improve sealing performance between the sealing bottle 246 and the second sealing member 247. In addition, when the sealing bottle 246 is detached from the needle base 241, the second sealing member 247 may be detached from the sensor base 245 with the sealing bottle 246.

In some embodiments, at least a part of the sealing bottle 246 allows ionizing radiation to pass through. For example, a part or all of regions of the sealing bottle 246 may allow ionizing radiation to pass through. The sealing bottle 246 may be made of engineering plastic such as polymethyl methacrylate and polycarbonate, and the engineering plastic such as methyl methacrylate and polycarbonate can both allow ionizing radiation to pass through.

Ionizing radiation may be one or a combination of electron beam radiation, gamma-ray radiation, and X-ray radiation. Ionizing radiation (as shown by an arrow in FIG. 32) passes through the sealing bottle 246, to sterilize the puncture tip 2421 of the puncture needle 242 and the implantation portion 2443 of the sensor 244. When irradiation sterilization is performed on the sensor sealing component 24, the sensor sealing component 24 is placed in a positioning structure of a tray, the positioning structure of the tray may be arranged in an array, a plurality of sensor sealing assemblies 24 may be placed on one tray, and the tray is placed in a sterilization room for irradiation sterilization.

In the conventional technology, when radiation sterilization is performed on an assembled sensor component or physiological sign detection apparatus, a dedicated sterilization apparatus needs to be customized to implement fixed-point sterilization on a puncture tip of a puncture needle and an implantation portion of a sensor. Fixed-point sterilization has a high requirement on a radiation device, and is difficult to be implemented in batched. In addition, when irradiation sterilization is performed on the entire sensor component or the entire physiological sign detection apparatus, the sensor component or the physiological sign detection apparatus needs to be assembled with the specific tooling, to perform shielding protection on an internal circuit of the sensor component. Consequently, sterilization procedures are cumbersome and inefficient.

In this embodiment, at least the partial region of the sealing bottle 246 allows ionizing radiation to pass through, so that the sensor sealing component 24 can sterilize the puncture tip 2421 of the puncture needle 242 and the implantation portion 2443 of the sensor 244 through irradiation. In addition, after sterilization, the puncture tip 2421 of the puncture needle 242 and the implantation portion 2443 of the sensor 244 are still located in the sealed space formed by using the sensor sealing component 24. As a sterile barrier, the sealing bottle 246 may form good isolation protection for the puncture tip 2421 of the puncture needle 242 and the implantation portion 2443 of the sensor 244. The puncture tip 2421 of the puncture needle 242 and the implantation portion 2443 of the sensor 244 are not contaminated for a second time. In subsequent packaging, a sterilized package for ensuring that the sensor 244 is located in a sterile environment does not need to be additionally added to the physiological sign detection apparatus 100, to reduce costs. In addition, because the sensor sealing component 24 may be independent of an internal circuit of the sensor component 20, it is possible to perform only radiation sterilization on the sensor sealing component 24, and no additional shielding protection needs to be set. Sterilization procedures are simple, and an internal circuit of the sensor 244 is not damaged. In addition, the sensor sealing component 24 has a small volume, and a plurality of sensor sealing assemblies 24 can be sterilized simultaneously through one radiation sterilization procedure, to greatly improve sterilization efficiency and reducing sterilization costs.

Refer to FIG. 2, FIG. 29B, and FIG. 33. In some embodiments, the implanter 10 is provided with a hook (not shown in FIG. 2), and the hook of the implanter 10 is snap-fitted into the snap-fitting portion 2412 of the needle base 241. The implanter 10 is configured to push the sensor component 20 to move toward a position at which the organism 30 is located, so that the bonding member 27 of the sensor component 20 can be stuck on the organism. After the sensor 244 is implanted into the organism 30 under guidance of the puncture needle 242, the hook of the implanter 10 drives, by using the needle base 241, the puncture needle 242 to move in a direction away from the organism 30, so that the puncture needle 242 is detached from the organism.

It may be understood that the sensor component 20 in the foregoing other embodiments may include the sensor sealing component 24 in this embodiment. In addition, other implementation structures of the sensor component 20 including the sensor sealing component 24 in this embodiment may also be of a plurality of types. The sensor component 20 may be used in various types of implanters.

The foregoing embodiments are merely used to describe the technical solutions of this application, but are not intended to limit the technical solutions. Although this application is described in detail with reference to the foregoing embodiments, persons of ordinary skill in the art should understand that they may still make modifications to the technical solutions described in the foregoing embodiments or make equivalent replacements to some technical features thereof, without departing from the scope of the technical solutions of embodiments of this application.

## Claims

1. A sensor sealing component, comprising:
a needle base;
a puncture needle, fastened to the needle base, wherein a puncture tip of the puncture needle protrudes from the needle base;
a sensor, comprising an implantation portion, wherein the implantation portion of the sensor is located on an inner side of the puncture needle; and
a sealing bottle, detachably fastened to the needle base, and hermetically connected to the needle base, wherein the sealing bottle has a hollow cavity, and the implantation portion of the sensor and the puncture tip of the puncture needle are located in the hollow cavity of the sealing bottle.

2. The sensor sealing component according to claim 1, wherein at least a partial region of the sealing bottle allows ionizing radiation to pass through.

3. The sensor sealing component according to claim 1 or 2, wherein the sealing bottle is threadedly connected to, snap-fitted into, or interference-connected to the needle base.

4. The sensor sealing component according to claim 1 or 2, wherein the sensor sealing component further comprises a snap-fit, the snap-fit is fastened to the needle base, and the sealing bottle is snap-fitted into or interference-connected to the snap-fit.

5. The sensor sealing component according to claim 4, wherein the sealing bottle comprises a boss, the boss is located at an end that is of the sealing bottle and that faces away from the needle base, and the sealing bottle is detached from the needle base when the boss is subject to an action force away from the needle base.

6. The sensor sealing component according to claim 1 or 2, wherein an inner side of the sealing bottle is provided with an internal thread, an outer side of the needle base is provided with an external thread, the internal thread is engaged with the external thread, the sealing bottle further comprises a ratchet, the ratchet is convexly disposed on an outer side surface of the sealing bottle, and the sealing bottle is detached from the needle base when the ratchet is subject to an action force opposite to a rotation direction of the internal thread of the sealing bottle.

7. The sensor sealing component according to any one of claims 1 to 6, wherein the sensor sealing component further comprises a sensor base, the sensor is fastened to the sensor base, the sensor base is located between the needle base and the sealing bottle, an end of the needle base passes through the sensor base, the sealing bottle is hermetically connected to the sensor base, and the needle base is hermetically connected to the sensor base.

8. The sensor sealing component according to claim 7, wherein the sensor and the sensor base are integrally formed by using an in-mold injection molding process, and a region that is of the sensor and that is in contact with the sensor base is covered with a heat insulation layer.

9. The sensor sealing component according to claim 7 or 8, wherein the sensor sealing component further comprises a first sealing member and a second sealing member, the first sealing member is hermetically connected to the needle base and the sensor base, and the second sealing member is hermetically connected to the sensor base and the sealing bottle.

10. The sensor sealing component according to any one of claims 1 to 6, wherein the sensor sealing component further comprises an upper cover, the sensor is snap-fitted into the upper cover, the upper cover is located between the needle base and the sealing bottle, an end of the needle base passes through the upper cover, the needle base is hermetically connected to the upper cover, and the sealing bottle is hermetically connected to the upper cover.

11. The sensor sealing component according to claim 10, wherein the upper cover has a second through hole, the upper cover comprises a blocking flange, the blocking flange is disposed around the second through hole, and the sensor is stuck on the blocking flange.

12. The sensor sealing component according to claim 11, wherein the upper cover further comprises a first positioning member, the first positioning member is spaced apart from the blocking flange, and the sensor is snap-fitted into the first positioning member.

13. A sensor component, comprising an upper cover, a lower cover, and the sensor sealing component according to any one of claims 1 to 9, wherein the upper cover is fastened to the lower cover, the lower cover has a first through hole, the upper cover has a second through hole, the second through hole directly faces and communicates with the first through hole, and the sensor sealing component runs through the first through hole and the second through hole, and is snap-fitted into the lower cover and/or the upper cover.

14. The sensor component according to claim 13, wherein the sensor component further comprises a battery, a connector, and a circuit board, accommodation space is formed between the upper cover and the lower cover, the circuit board, the connector, the battery, and a connection portion of the sensor is located in the accommodation space, the circuit board is fastened to the lower cover or the upper cover, the battery and the connector are fastened to the circuit board and are electrically connected to the circuit board, and the sensor of the sensor sealing component is electrically connected to the connector.

15. A sensor component, comprising a circuit board, a connector, and the sensor sealing component according to any one of claims 1 to 6 and 10 to 12, wherein the circuit board is arranged on a circumferential side of the sensor sealing component, the connector is fastened to the circuit board and is electrically connected to the circuit board, and a sensor of the sensor sealing component is electrically connected to the connector.

16. The sensor component according to claim 14 or 15, wherein the connector comprises a connecting groove, a plurality of first spring plates, and a plurality of second spring plates, the connecting groove comprises a first groove wall and a second groove wall that are disposed opposite to each other, the plurality of first spring plates are convexly disposed on the first groove wall, the plurality of second spring plates are convexly disposed on the second groove wall, and a connection portion of the sensor is snap-fitted into the connecting groove of the connector and is electrically connected to the plurality of first spring plates and/or the plurality of second spring plates.

17. The sensor component according to claim 16, wherein the first spring plate and the second spring plate each are formed by bending a metal plate, a plate surface of the first spring plate is in contact with the sensor, and a plate surface of the second spring plate is in contact with the sensor.

18. A physiological sign detection apparatus, comprising an implanter and the sensor component according to any one of claims 13 to 17, wherein the sensor component is detachably fastened to the implanter, and the implanter is configured to push the sensor component to an organism.
